# EUROPEAN PATENT SPECIFICATION

(11) **EP 2 960 326 B1**
(45) Date of publication and mention of the grant of the patent: **03.04.2019**
(21) Application number: 15739495.8
(22) Date of filing: 08.05.2015
(51) Int. Cl.: C12N 1/19, C12N 15/52, C12N 1/18, C12N 9/02, C12N 9/04, C12N 9/88, C12P 7/56, C12R 1/865

(54) **MICROORGANISM CAPABLE OF ENHANCING LACTIC ACID PRODUCTION AND METHOD FOR PRODUCING LACTIC ACID USING SAME**
MIKROORGANISMUS, DER ZUR ERHÖHTEN MILCHSÄUREHERSTELLUNG BEFÄHIGT IST, UND VERFAHREN ZUR HERSTELLUNG VON MILCHSÄURE UNTER DESSEN VERWENDUNG
MICROORGANISME POUVANT AMÉLIORER LA PRODUCTION D'ACIDE LACTIQUE ET PROCÉDÉ DE PRODUCTION D'ACIDE LACTIQUE L'UTILISANT

(30) Priority: 09.05.2014 KR 20140055865
(43) Date of publication of application: 30.12.2015
(73) Proprietor: Cj Cheiljedang Corporation, Seoul 100-400 (KR)
(72) Inventor: YANG, Eun Bin, Seoul 156-070 (KR); LEE, Tae Hee, Seongnam-si Gyeonggi-do 463-921 (KR); KIM, Seon Hye, Suwon-si Gyeonggi-do 443-757 (KR); SONG, Gyu Hyeon, Seoul 157-840 (KR); HA, Cheol Woong, Seoul 157-742 (KR); NA, Kyung Su, Gimpo-si Gyeonggi-do 415-759 (KR); YANG, Young Lyeol, Seoul 135-806 (KR); KANG, Min Sun, Seoul 157-839 (KR); LEE, Hyo Hyoung, Incheon 404-775 (KR)
(74) Representative: Schiweck Weinzierl Koch Patentanwälte Partnerschaft mbB
(86) International application number: PCT/KR2015/004600
(87) International publication number: WO 2015/170914

(56) References cited:
- EP-A1- 2 281 881
- EP-A2- 2 314 638
- WO-A1-99/14335
- WO-A1-2013/052604
- WO-A2-2011/161317
- JP-A- 2011 254 747
- KR-A- 20000 067 653
- KR-A- 20020 048 910
- KR-A- 20130 098 603
- KR-A- 20130 126 809
- KR-A- 20140 001 165
- KANOKARN KOCHARIN ET AL: "Engineering of acetyl-CoA metabolism for the improved production of polyhydroxybutyrate in Saccharomyces cerevisiae", AMB EXPRESS, vol. 2, no. 1, 25 September 2012 (2012-09-25), page 52, XP55309384, DE ISSN: 2191-0855, DOI: 10.1128/AEM.72.1.536-543.2006
- SHIBA ET AL: "Engineering of the pyruvate dehydrogenase bypass in Saccharomyces cerevisiae for high-level production of isoprenoids", METABOLIC ENGINEERING, ACADEMIC PRESS, US, vol. 9, no. 2, 17 November 2006 (2006-11-17), pages 160-168, XP005888123, ISSN: 1096-7176, DOI: 10.1016/J.YMBEN.2006.10.005
- Nobuhiro Ishida ET AL: "Metabolic Engineering of Saccharomyces cerevisiae for Efficient Production of Pure L-(+)-Lactic Acid" In: "Twenty-Seventh Symposium on Biotechnology for Fuels and Chemicals", 2006, Humana Press, Totowa, NJ, XP55177640, ISBN: 978-1-58-829866-9 vol. 795, pages 795-807, DOI: 10.1007/978-1-59745-268-7_65, * table 2 *
- ANASTASIA KRIVORUCHKO ET AL: "Improving biobutanol production in engineered Saccharomyces cerevisiae by manipulation of acetyl-CoA metabolism", JOURNAL OF INDUSTRIAL MICROBIOLOGY & BIOTECHNOLOGY, vol. 40, no. 9, 13 June 2013 (2013-06-13), pages 1051-1056, XP55087006, ISSN: 1367-5435, DOI: 10.1007/s10295-013-1296-0
- NOBUHIRO ISHIDA ET AL: "Efficient production of L-lactic acid by metabolically engineered saccharomyces cerevisiae with a genome-integrated L-lactate dehydrogenase gene", APPLIED AND ENVIRONMENTAL MICROBIOLOGY, AMERICAN SOCIETY FOR MICROBIOLOGY, US, vol. 71, no. 4, April 2005 (2005-04), pages 1964-1970, XP008128665, ISSN: 0099-2240, DOI: 10.1128/AEM:71.4.1964-1970.2005
- ISHIDA N ET AL: "d-Lactic acid production by metabolically engineered Saccharomyces cerevisiae", JOURNAL OF BIOSCIENCE AND BIOENGINEERING, ELSEVIER, AMSTERDAM, NL, vol. 101, no. 2, 28 March 2006 (2006-03-28), pages 172-177, XP028042262, ISSN: 1389-1723, DOI: 10.1263/JBB.101.172 [retrieved on 2006-02-01]
- AKAMATSU S1 ET AL: "Effects of aldehyde dehydrogenase and acetyl-CoA synthetase on acetate formation in sake mash", JOURNAL OF BIOSCIENCE AND BIOENGINEERING, ELSEVIER, AMSTERDAM, NL, vol. 90, no. 5, 26 January 2001 (2001-01-26), pages 555-560, XP002720404, ISSN: 1389-1723, DOI: 10.1016/S1389-1723(01)80040-2
- TOKUHIRO, KENRO. ET AL.: 'Double mutation of the PDC1 and ADH1 genes improves lactate production in the yeast Saccharomyces cerevisiae expressing the bovine lactate dehydrogenase gene .' APPL MICROBIOL BIOTECHNOL. vol. 82, 2009, pages 883 - 890, XP019705475
- ISHIDA, NOBUHIRO. ET AL.: 'The Effect of Pyruvate Decarboxylase Gene Knockout in Saccharomyces cerevisiae on L-Lactic Acid Production.' BIOSCI BIOTECHNOL BIOCHEM vol. 70, no. 5, 2006, pages 1148 - 1153, XP008129166
- BIANCHI, M. MICHELE. ET AL.: 'Efficient Homolactic Fermentation by Kluyveromyces lactis Strains Defective in Pyruvate Utilization and Transformed with the Heterologous LDH Gene .' APPL ENVIRON MICROBIOL. vol. 67, no. 12, 2001, pages 5621 - 5625, XP002236026
- PING LIU ET AL.: 'Metabolic engineering of biocatalysts for carboxylic acids production.' CSBJ. vol. 3, no. ISSUE,, 2012, pages 1 - 9, XP055177846

<110> CJ CheilJedang Corporation
<120> A microorganism having improved productivity of lactic acid and a process for producing lactic acid using the same
<130> OPA15034
<150> KR10-2014-0055865
   <151> 2014-05-09
<160> 78
<170> KopatentIn 2.0
<210> 1
   <211> 32
   <212> DNA
   <213> Artificial Sequence
<220>
   <223> ADH1 upstream forward primer
<400> 1
   cgggatccac tgtagcccta gacttgatag cc 32
<210> 2
   <211> 44
   <212> DNA
   <213> Artificial Sequence
<220>
   <223> ADH1 upstream reverse primer
<400> 2
   ataagaatgc ggccgctgta tatgagatag ttgattgtat gctt 44
<210> 3
   <211> 37
   <212> DNA
   <213> Artificial Sequence
<220>
   <223> ADH1 downstream forward primer
<400> 3
   gactagtgcg aatttcttat gatttatgat ttttatt 37
<210> 4
   <211> 37
   <212> DNA
   <213> Artificial Sequence
<220>
   <223> ADH1 downstream reverse primer
<400> 4
   acatgccatg gaagcatgca cgtatacact tgagtaa 37
<210> 5
   <211> 35
   <212> DNA
   <213> Artificial Sequence
<220>
   <223> PDC1 upstream forward primer
<400> 5
   cgggatccat tatgtatgct cttctgactt ttcgt 35
<210> 6
   <211> 44
   <212> DNA
   <213> Artificial Sequence
<220>
   <223> PDC1 upstream reverse primer
<400> 6
   ataagaatgc ggccgctttg attgatttga ctgtgttatt ttgc 44
<210> 7
   <211> 37
   <212> DNA
   <213> Artificial Sequence
<220>
   <223> PDC1 downstream forward primer
<400> 7
   cgggatccgc gatttaatct ctaattatta gttaaag 37
<210> 8
   <211> 44
   <212> DNA
   <213> Artificial Sequence
<220>
   <223> PDC1 downstream reverse primer
<400> 8
   ataagaatgc ggccgctttc aatcattgga gcaatcattt taca 44
<210> 9
   <211> 50
   <212> DNA
   <213> Artificial Sequence
<220>
   <223> DLD1-HIS3 upstream linking primer
<400> 9
   gcgtagttgg ccccaactgg tgcagtaata cgttttaaga gcttggtgag 50
<210> 10
   <211> 50
   <212> DNA
   <213> Artificial Sequence
<220>
   <223> DLD1-HIS3 downstream linking primer
<400> 10
   cgtgaagggt gaaaaaggaa aatcagatac ctacataaga acacctttgg 50
<210> 11
   <211> 22
   <212> DNA
   <213> Artificial Sequence
<220>
   <223> F_PDC5_UP_676
<400> 11
   gtcagcattg acacgttcga tt 22
<210> 12
   <211> 39
   <212> DNA
   <213> Artificial Sequence
<220>
   <223> R_K1URA3-PDC5_UP
<400> 12
   tctacccaga atcacttctt tcgagagatt gtcataatc 39
<210> 13
   <211> 38
   <212> DNA
   <213> Artificial Sequence
<220>
   <223> F_PDC5_UP-AL_K1URA3
<400> 13
   caatctctcg aaagaagtga ttctgggtag aagatcgg 38
<210> 14
   <211> 27
   <212> DNA
   <213> Artificial Sequence
<220>
   <223> R_AL_K1URA3
<400> 14
   gagcaatgaa cccaataacg aaatctt 27
<210> 15
   <211> 24
   <212> DNA
   <213> Artificial Sequence
<220>
   <223> F_BR_KlURA3
<400> 15
   cttgacgttc gttcgactga tgag 24
<210> 16
   <211> 22
   <212> DNA
   <213> Artificial Sequence
<220>
   <223> R_PDC5_DOWN_522
<400> 16
   caagtcaacc aagttagctg gc 22
<210> 17
   <211> 43
   <212> DNA
   <213> Artificial Sequence
<220>
   <223> R_SCO1p-BR_KlURA3
<400> 17
   ctctcctaat agacgtggtg tcaccatgaa cgacaattct taa 43
<210> 18
   <211> 38
   <212> DNA
   <213> Artificial Sequence
<220>
   <223> F_SCO1p_500
<400> 18
   cgttcatggt gacaccacgt ctattaggag agccattc 38
<210> 19
   <211> 41
   <212> DNA
   <213> Artificial Sequence
<220>
   <223> R_PDC5_DOWN_500-SCO1p
<400> 19
   aaggttattt cagacatctt ttctacgttt gctgtttttt c 41
<210> 20
   <211> 42
   <212> DNA
   <213> Artificial Sequence
<220>
   <223> F_SCO1p-PDC5_DOWN_500
<400> 20
   cagcaaacgt agaaaagatg tctgaaataa ccttaggtaa at 42
<210> 21
   <211> 44
   <212> DNA
   <213> Artificial Sequence
<220>
   <223> R_SC02p-BR_KlURA3
<400> 21
   atcgaataag taacaagcgt gtcaccatga acgacaattc ttaa 44
<210> 22
   <211> 38
   <212> DNA
   <213> Artificial Sequence
<220>
   <223> F_SC02p_500
<400> 22
   cgttcatggt gacacgcttg ttacttattc gataacgc 38
<210> 23
   <211> 40
   <212> DNA
   <213> Artificial Sequence
<220>
   <223> R_PDC5_DOWN_500-SC02p
<400> 23
   aaggttattt cagacatttt actctcgctt cccaaattcc 40
<210> 24
   <211> 40
   <212> DNA
   <213> Artificial Sequence
<220>
   <223> F_SCO2p-PDC5_DOWN_500
<400> 24
   ggaagcgaga gtaaaatgtc tgaaataacc ttaggtaaat 40
<210> 25
   <211> 47
   <212> DNA
   <213> Artificial Sequence
<220>
   <223> R_IDP2p-BR_KlURA3
<400> 25
   taaaaataaa tagatagacg tgtgtcacca tgaacgacaa ttcttaa 47
<210> 26
   <211> 42
   <212> DNA
   <213> Artificial Sequence
<220>
   <223> F_IDP2p_500
<400> 26
   cgttcatggt gacacacgtc tatctattta tttttataac tc 42
<210> 27
   <211> 44
   <212> DNA
   <213> Artificial Sequence
<220>
   <223> R_PDC5_DOWN_500-IDP2p
<400> 27
   aaggttattt cagacattac gattttatat atatacgtac gtta 44
<210> 28
   <211> 45
   <212> DNA
   <213> Artificial Sequence
<220>
   <223> F_IDP2p-PDC5_DOWN_500
<400> 28
   cgtatatata taaaatcgta atgtctgaaa taaccttagg taaat 45
<210> 29
   <211> 43
   <212> DNA
   <213> Artificial Sequence
<220>
   <223> R_ACS1p-BR_KlURA3
<400> 29
   ctggacgtat gtgcacagtg tcaccatgaa cgacaattct taa 43
<210> 30
   <211> 38
   <212> DNA
   <213> Artificial Sequence
<220>
   <223> F_ACS1p_500
<400> 30
   cgttcatggt gacactgtgc acatacgtcc agaatgat 38
<210> 31
   <211> 39
   <212> DNA
   <213> Artificial Sequence
<220>
   <223> R_PDC5_DOWN_500-ACS1p
<400> 31
   aaggttattt cagacatagc acagtgggca atgtctttc 39
<210> 32
   <211> 41
   <212> DNA
   <213> Artificial Sequence
<220>
   <223> F_ACS1p-PDC5_DOWN_500
<400> 32
   cattgcccac tgtgctatgt ctgaaataac cttaggtaaa t 41
<210> 33
   <211> 45
   <212> DNA
   <213> Artificial Sequence
<220>
   <223> R_FBA1p-BR_KlURA3
<400> 33
   ttatttacgt aatgacccag tgtcaccatg aacgacaatt cttaa 45
<210> 34
   <211> 40
   <212> DNA
   <213> Artificial Sequence
<220>
   <223> F_FBA1p_500
<400> 34
   cgttcatggt gacactgggt cattacgtaa ataatgatag 40
<210> 35
   <211> 44
   <212> DNA
   <213> Artificial Sequence
<220>
   <223> R_PDC5_DOWN_500-FBA1p
<400> 35
   aaggttattt cagacatttt gaatatgtat tacttggtta tggt 44
<210> 36
   <211> 45
   <212> DNA
   <213> Artificial Sequence
<220>
   <223> F_FBA1p-PDC5_DOWN_500
<400> 36
   ccaagtaata catattcaaa atgtctgaaa taaccttagg taaat 45
<210> 37
   <211> 41
   <212> DNA
   <213> Artificial Sequence
<220>
   <223> F-ALPDC5-BamHI
<400> 37
   gagctcggat ccaaggaaat aaagcaaata acaataacac c 41
<210> 38
   <211> 43
   <212> DNA
   <213> Artificial Sequence
<220>
   <223> R-ALPDC5-NotI
<400> 38
   accatggcgg ccgctttgtt cttcttgtta ttgtattgtg ttg 43
<210> 39
   <211> 42
   <212> DNA
   <213> Artificial Sequence
<220>
   <223> F-BRPDC5-SpeI
<400> 39
   ggatccacta gtgctaatta acataaaact catgattcaa cg 42
<210> 40
   <211> 42
   <212> DNA
   <213> Artificial Sequence
<220>
   <223> R-BRPDC5-NcoI
<400> 40
   cagctgccat ggtattctaa ataagatgta aggccttgta at 42
<210> 41
   <211> 38
   <212> DNA
   <213> Artificial Sequence
<220>
   <223> F_PDC1
<400> 41
   ataactagta tgtctgaaat tactttgggt aaatattt 38
<210> 42
   <211> 44
   <212> DNA
   <213> Artificial Sequence
<220>
   <223> R_PDC1
<400> 42
   caaaggaaaa ggggcctgtt tattgcttag cgttggtagc agca 44
<210> 43
   <211> 43
   <212> DNA
   <213> Artificial Sequence
<220>
   <223> F_CYC1t
<400> 43
   taccaacgct aagcaataaa caggcccctt ttcctttgtc gat 43
<210> 44
   <211> 33
   <212> DNA
   <213> Artificial Sequence
<220>
   <223> R_CYC1t
<400> 44
   atactcgagg caaattaaag ccttcgagcg tcc 33
<210> 45
   <211> 33
   <212> DNA
   <213> Artificial Sequence
<220>
   <223> F_PDC1p
<400> 45
   aaagagctcc atgcgactgg gtgagcatat gtt 33
<210> 46
   <211> 37
   <212> DNA
   <213> Artificial Sequence
<220>
   <223> R_PDC1p
<400> 46
   ataactagtt ttgattgatt tgactgtgtt attttgc 37
<210> 47
   <211> 37
   <212> DNA
   <213> Artificial Sequence
<220>
   <223> F_IDP2p
<400> 47
   aaagagctca cgtctatcta tttattttta taactcc 37
<210> 48
   <211> 37
   <212> DNA
   <213> Artificial Sequence
<220>
   <223> R_IDP2p
<400> 48
   ataactagtt acgattttat atatatacgt acgttac 37
<210> 49
   <211> 35
   <212> DNA
   <213> Artificial Sequence
<220>
   <223> F_BamHI-PDC5_UP
<400> 49
   cgggatccag gccaaggaaa taaagcaaat aacaa 35
<210> 50
   <211> 44
   <212> DNA
   <213> Artificial Sequence
<220>
   <223> R_NotI-PDC5_UP
<400> 50
   ataagaatgc ggccgctttg ttcttcttgt tattgtattg tgtt 44
<210> 51
   <211> 36
   <212> DNA
   <213> Artificial Sequence
<220>
   <223> F_BamHI-PDC5_DOWN
<400> 51
   cgggatccgc taattaacat aaaactcatg attcaa 36
<210> 52
   <211> 44
   <212> DNA
   <213> Artificial Sequence
<220>
   <223> R_NotI-PDC5_DOWN
<400> 52
   ataagaatgc ggccgctatt ctaaataaga tgtaaggcct tgta 44
<210> 53
   <211> 27
   <212> DNA
   <213> Artificial Sequence
<220>
   <223> F_PDC5_UP
<400> 53
   aggccaagga aataaagcaa ataacaa 27
<210> 54
   <211> 27
   <212> DNA
   <213> Artificial Sequence
<220>
   <223> R_AL_KlURA3
<400> 54
   gagcaatgaa cccaataacg aaatctt 27
<210> 55
   <211> 24
   <212> DNA
   <213> Artificial Sequence
<220>
   <223> F_BR_KlURA3
<400> 55
   cttgacgttc gttcgactga tgag 24
<210> 56
   <211> 28
   <212> DNA
   <213> Artificial Sequence
<220>
   <223> R_PDC5_DOWN
<400> 56
   tattctaaat aagatgtaag gccttgta 28
<210> 57
   <211> 35
   <212> DNA
   <213> Artificial Sequence
<220>
   <223> F_BamHI-PDC6_UP
<400> 57
   cgggatcctg ttatagagtt cacaccttat tcaca 35
<210> 58
   <211> 45
   <212> DNA
   <213> Artificial Sequence
<220>
   <223> R_NotI-PDC6_UP
<400> 58
   ataagaatgc ggccgctttg ttggcaatat gtttttgcta tatta 45
<210> 59
   <211> 34
   <212> DNA
   <213> Artificial Sequence
<220>
   <223> F_BamHI-PDC6_DOWN
<400> 59
   cgggatccgc cattagtagt gtactcaaac gaat 34
<210> 60
   <211> 43
   <212> DNA
   <213> Artificial Sequence
<220>
   <223> R_NotI-PDC6_DOWN
<400> 60
   ataagaatgc ggccgcgatg cagaatgagc acttgttatt tat 43
<210> 61
   <211> 27
   <212> DNA
   <213> Artificial Sequence
<220>
   <223> F_PDC6_UP
<400> 61
   tgttatagag ttcacacctt attcaca 27
<210> 62
   <211> 27
   <212> DNA
   <213> Artificial Sequence
<220>
   <223> R_AL_KlURA3
<400> 62
   gagcaatgaa cccaataacg aaatctt 27
<210> 63
   <211> 24
   <212> DNA
   <213> Artificial Sequence
<220>
   <223> F_BR_KlURA3
<400> 63
   cttgacgttc gttcgactga tgag 24
<210> 64
   <211> 27
   <212> DNA
   <213> Artificial Sequence
<220>
   <223> R_PDC6_DOWN
<400> 64
   gatgcagaat gagcacttgt tatttat 27
<210> 65
   <211> 50
   <212> DNA
   <213> Artificial Sequence
<220>
   <223> F_SpeI_ALD2
<400> 65
   caagctggcc gctctagaac tagtatgcct accttgtata ctgatatcga 50
<210> 66
   <211> 50
   <212> DNA
   <213> Artificial Sequence
<220>
   <223> R_XhoI_ALD2
<400> 66
   acataactaa ttacatgact cgagttagtt gtccaaagag agatttatgt 50
<210> 67
   <211> 50
   <212> DNA
   <213> Artificial Sequence
<220>
   <223> F_SpeI_ALD3
<400> 67
   caagctggcc gctctagaac tagtatgcct accttgtata ctgatatcga 50
<210> 68
   <211> 50
   <212> DNA
   <213> Artificial Sequence
<220>
   <223> R_XhoI_ALD3
<400> 68
   acataactaa ttacatgact cgagttattt atccaatgaa agatccacat 50
<210> 69
   <211> 46
   <212> DNA
   <213> Artificial Sequence
<220>
   <223> F_SpeI_ACS1
<400> 69
   tccaagctgg ccgctctaga actagtatgt cgccctctgc cgtaca 46
<210> 70
   <211> 50
   <212> DNA
   <213> Artificial Sequence
<220>
   <223> R_EcoRI_ACS1
<400> 70
   tatcgataag cttgatatcg aattcttaca acttgaccga atcaattaga 50
<210> 71
   <211> 563
   <212> PRT
   <213> Saccharomyces cerevisiae PDC1
<400> 71
   Met Ser Glu Ile Thr Leu Gly Lys Tyr Leu Phe Glu Arg Leu Lys Gln
   1               5                   10                  15
   Val Asn Val Asn Thr Val Phe Gly Leu Pro Gly Asp Phe Asn Leu Ser
               20                  25                  30
   Leu Leu Asp Lys Ile Tyr Glu Val Glu Gly Met Arg Trp Ala Gly Asn
           35                  40                  45
   Ala Asn Glu Leu Asn Ala Ala Tyr Ala Ala Asp Gly Tyr Ala Arg Ile
       50                  55                  60
   Lys Gly Met Ser Cys Ile Ile Thr Thr Phe Gly Val Gly Glu Leu Ser
   65                  70                  75                  80
   Ala Leu Asn Gly Ile Ala Gly Ser Tyr Ala Glu His Val Gly Val Leu
                   85                  90                  95
   His Val Val Gly Val Pro Ser Ile Ser Ala Gln Ala Lys Gln Leu Leu
               100                 105                 110
   Leu His His Thr Leu Gly Asn Gly Asp Phe Thr Val Phe His Arg Met
           115                 120                 125
   Ser Ala Asn Ile Ser Glu Thr Thr Ala Met Ile Thr Asp Ile Ala Thr
       130                 135                 140
   Ala Pro Ala Glu Ile Asp Arg Cys Ile Arg Thr Thr Tyr Val Thr Gln
   145                 150                 155                 160
   Arg Pro Val Tyr Leu Gly Leu Pro Ala Asn Leu Val Asp Leu Asn Val
                   165                 170                 175
   Pro Ala Lys Leu Leu Gln Thr Pro Ile Asp Met Ser Leu Lys Pro Asn
               180                 185                 190
   Asp Ala Glu Ser Glu Lys Glu Val Ile Asp Thr Ile Leu Ala Leu Val
           195                 200                 205
   Lys Asp Ala Lys Asn Pro Val Ile Leu Ala Asp Ala Cys Cys Ser Arg
       210                 215                 220
   His Asp Val Lys Ala Glu Thr Lys Lys Leu Ile Asp Leu Thr Gln Phe
   225                 230                 235                 240
   Pro Ala Phe Val Thr Pro Met Gly Lys Gly Ser Ile Asp Glu Gln His
                   245                 250                 255
   Pro Arg Tyr Gly Gly Val Tyr Val Gly Thr Leu Ser Lys Pro Glu Val
               260                 265                 270
   Lys Glu Ala Val Glu Ser Ala Asp Leu Ile Leu Ser Val Gly Ala Leu
           275                 280                 285
   Leu Ser Asp Phe Asn Thr Gly Ser Phe Ser Tyr Ser Tyr Lys Thr Lys
       290                 295                 300
   Asn Ile Val Glu Phe His Ser Asp His Met Lys Ile Arg Asn Ala Thr
   305                 310                 315                 320
   Phe Pro Gly Val Gln Met Lys Phe Val Leu Gln Lys Leu Leu Thr Thr
                   325                 330                 335
   Ile Ala Asp Ala Ala Lys Gly Tyr Lys Pro Val Ala Val Pro Ala Arg
               340                 345                 350
   Thr Pro Ala Asn Ala Ala Val Pro Ala Ser Thr Pro Leu Lys Gln Glu
           355                 360                 365
   Trp Met Trp Asn Gln Leu Gly Asn Phe Leu Gln Glu Gly Asp Val Val
       370                 375                 380
   Ile Ala Glu Thr Gly Thr Ser Ala Phe Gly Ile Asn Gln Thr Thr Phe
   385                 390                 395                 400
   Pro Asn Asn Thr Tyr Gly Ile Ser Gln Val Leu Trp Gly Ser Ile Gly
                   405                 410                 415
   Phe Thr Thr Gly Ala Thr Leu Gly Ala Ala Phe Ala Ala Glu Glu Ile
               420                 425                 430
   Asp Pro Lys Lys Arg Val Ile Leu Phe Ile Gly Asp Gly Ser Leu Gln
           435                 440                 445
   Leu Thr Val Gln Glu Ile Ser Thr Met Ile Arg Trp Gly Leu Lys Pro
       450                 455                 460
   Tyr Leu Phe Val Leu Asn Asn Asp Gly Tyr Thr Ile Glu Lys Leu Ile
   465                 470                 475                 480
   His Gly Pro Lys Ala Gln Tyr Asn Glu Ile Gln Gly Trp Asp His Leu
                   485                 490                 495
   Ser Leu Leu Pro Thr Phe Gly Ala Lys Asp Tyr Glu Thr His Arg Val
               500                 505                 510
   Ala Thr Thr Gly Glu Trp Asp Lys Leu Thr Gln Asp Lys Ser Phe Asn
           515                 520                 525
   Asp Asn Ser Lys Ile Arg Met Ile Glu Ile Met Leu Pro Val Phe Asp
       530                 535                 540
   Ala Pro Gln Asn Leu Val Glu Gln Ala Lys Leu Thr Ala Ala Thr Asn
   545                 550                 555                 560
   Ala Lys Gln
<210> 72
   <211> 563
   <212> PRT
   <213> Saccharomyces cerevisiae PDC5
<400> 72
   Met Ser Glu Ile Thr Leu Gly Lys Tyr Leu Phe Glu Arg Leu Ser Gln
    1               5                  10                  15
   Val Asn Cys Asn Thr Val Phe Gly Leu Pro Gly Asp Phe Asn Leu Ser
               20                  25                  30
   Leu Leu Asp Lys Leu Tyr Glu Val Lys Gly Met Arg Trp Ala Gly Asn
           35                  40                  45
   Ala Asn Glu Leu Asn Ala Ala Tyr Ala Ala Asp Gly Tyr Ala Arg Ile
       50                  55                  60
   Lys Gly Met Ser Cys Ile Ile Thr Thr Phe Gly Val Gly Glu Leu Ser
   65                  70                  75                  80
   Ala Leu Asn Gly Ile Ala Gly Ser Tyr Ala Glu His Val Gly Val Leu
                   85                  90                  95
   His Val Val Gly Val Pro Ser Ile Ser Ser Gln Ala Lys Gln Leu Leu
               100                 105                 110
   Leu His His Thr Leu Gly Asn Gly Asp Phe Thr Val Phe His Arg Met
           115                 120                 125
   Ser Ala Asn Ile Ser Glu Thr Thr Ala Met Ile Thr Asp Ile Ala Asn
       130                 135                 140
   Ala Pro Ala Glu Ile Asp Arg Cys Ile Arg Thr Thr Tyr Thr Thr Gln
   145 150 155 160
   Arg Pro Val Tyr Leu Gly Leu Pro Ala Asn Leu Val Asp Leu Asn Val
   165 170 175
   Pro Ala Lys Leu Leu Glu Thr Pro Ile Asp Leu Ser Leu Lys Pro Asn
   180 185 190
   Asp Ala Glu Ala Glu Ala Glu Val Val Arg Thr Val Val Glu Leu Ile
   195 200 205
   Lys Asp Ala Lys Asn Pro Val Ile Leu Ala Asp Ala Cys Ala Ser Arg
   210 215 220
   His Asp Val Lys Ala Glu Thr Lys Lys Leu Met Asp Leu Thr Gln Phe
   225 230 235 240
   Pro Val Tyr Val Thr Pro Met Gly Lys Gly Ala Ile Asp Glu Gln His
   245 250 255
   Pro Arg Tyr Gly Gly Val Tyr Val Gly Thr Leu Ser Arg Pro Glu Val
   260 265 270
   Lys Lys Ala Val Glu Ser Ala Asp Leu Ile Leu Ser Ile Gly Ala Leu
   275 280 285
   Leu Ser Asp Phe Asn Thr Gly Ser Phe Ser Tyr Ser Tyr Lys Thr Lys
   290 295 300
   Asn Ile Val Glu Phe His Ser Asp His Ile Lys Ile Arg Asn Ala Thr
   305 310 315 320
   Phe Pro Gly Val Gln Met Lys Phe Ala Leu Gln Lys Leu Leu Asp Ala
   325 330 335
   Ile Pro Glu Val Val Lys Asp Tyr Lys Pro Val Ala Val Pro Ala Arg
   340 345 350
   Val Pro Ile Thr Lys Ser Thr Pro Ala Asn Thr Pro Met Lys Gln Glu
   355 360 365
   Trp Met Trp Asn His Leu Gly Asn Phe Leu Arg Glu Gly Asp Ile Val
   370 375 380
   Ile Ala Glu Thr Gly Thr Ser Ala Phe Gly Ile Asn Gln Thr Thr Phe
   385 390 395 400
   Pro Thr Asp Val Tyr Ala Ile Val Gln Val Leu Trp Gly Ser Ile Gly
   405 410 415
   Phe Thr Val Gly Ala Leu Leu Gly Ala Thr Met Ala Ala Glu Glu Leu
   420 425 430
   Asp Pro Lys Lys Arg Val Ile Leu Phe Ile Gly Asp Gly Ser Leu Gln
   435 440 445
   Leu Thr Val Gln Glu Ile Ser Thr Met Ile Arg Trp Gly Leu Lys Pro
   450 455 460
   Tyr Ile Phe Val Leu Asn Asn Asn Gly Tyr Thr Ile Glu Lys Leu Ile
   465 470 475 480
   His Gly Pro His Ala Glu Tyr Asn Glu Ile Gln Gly Trp Asp His Leu
                   485                 490                 495
   Ala Leu Leu Pro Thr Phe Gly Ala Arg Asn Tyr Glu Thr His Arg Val
               500                 505                 510
   Ala Thr Thr Gly Glu Trp Glu Lys Leu Thr Gln Asp Lys Asp Phe Gln
           515                 520                 525
   Asp Asn Ser Lys Ile Arg Met Ile Glu Val Met Leu Pro Val Phe Asp
       530                 535                 540
   Ala Pro Gln Asn Leu Val Lys Gln Ala Gln Leu Thr Ala Ala Thr Asn
   545                 550                 555                 560
   Ala Lys Gln
<210> 73
   <211> 563
   <212> PRT
   <213> Saccharomyces cerevisiae PDC6
<400> 73
   Met Ser Glu Ile Thr Leu Gly Lys Tyr Leu Phe Glu Arg Leu Lys Gln
    1               5                  10                  15
   Val Asn Val Asn Thr Ile Phe Gly Leu Pro Gly Asp Phe Asn Leu Ser
               20                  25                  30
   Leu Leu Asp Lys Ile Tyr Glu Val Asp Gly Leu Arg Trp Ala Gly Asn
           35                  40                  45
   Ala Asn Glu Leu Asn Ala Ala Tyr Ala Ala Asp Gly Tyr Ala Arg Ile
       50                  55                  60
   Lys Gly Leu Ser Val Leu Val Thr Thr Phe Gly Val Gly Glu Leu Ser
   65                  70                  75                  80
   Ala Leu Asn Gly Ile Ala Gly Ser Tyr Ala Glu His Val Gly Val Leu
                   85                  90                  95
   His Val Val Gly Val Pro Ser Ile Ser Ala Gln Ala Lys Gln Leu Leu
               100                 105                 110
   Leu His His Thr Leu Gly Asn Gly Asp Phe Thr Val Phe His Arg Met
           115                 120                 125
   Ser Ala Asn Ile Ser Glu Thr Thr Ser Met Ile Thr Asp Ile Ala Thr
       130                 135                 140
   Ala Pro Ser Glu Ile Asp Arg Leu Ile Arg Thr Thr Phe Ile Thr Gln
   145                 150                 155                 160
   Arg Pro Ser Tyr Leu Gly Leu Pro Ala Asn Leu Val Asp Leu Lys Val
                   165                 170                 175
   Pro Gly Ser Leu Leu Glu Lys Pro Ile Asp Leu Ser Leu Lys Pro Asn
               180                 185                 190
   Asp Pro Glu Ala Glu Lys Glu Val Ile Asp Thr Val Leu Glu Leu Ile
               195                 200                 205
   Gln Asn Ser Lys Asn Pro Val Ile Leu Ser Asp Ala Cys Ala Ser Arg
       210                 215                 220
   His Asn Val Lys Lys Glu Thr Gln Lys Leu Ile Asp Leu Thr Gln Phe
   225                 230                 235                 240
   Pro Ala Phe Val Thr Pro Leu Gly Lys Gly Ser Ile Asp Glu Gln His
                   245                 250                 255
   Pro Arg Tyr Gly Gly Val Tyr Val Gly Thr Leu Ser Lys Gln Asp Val
               260                 265                 270
   Lys Gln Ala Val Glu Ser Ala Asp Leu Ile Leu Ser Val Gly Ala Leu
           275                 280                 285
   Leu Ser Asp Phe Asn Thr Gly Ser Phe Ser Tyr Ser Tyr Lys Thr Lys
       290                 295                 300
   Asn Val Val Glu Phe His Ser Asp Tyr Val Lys Val Lys Asn Ala Thr
   305                 310                 315                 320
   Phe Leu Gly Val Gln Met Lys Phe Ala Leu Gln Asn Leu Leu Lys Val
                   325                 330                 335
   Ile Pro Asp Val Val Lys Gly Tyr Lys Ser Val Pro Val Pro Thr Lys
               340                 345                 350
   Thr Pro Ala Asn Lys Gly Val Pro Ala Ser Thr Pro Leu Lys Gln Glu
           355                 360                 365
   Trp Leu Trp Asn Glu Leu Ser Lys Phe Leu Gln Glu Gly Asp Val Ile
       370                 375                 380
   Ile Ser Glu Thr Gly Thr Ser Ala Phe Gly Ile Asn Gln Thr Ile Phe
   385                 390                 395                 400
   Pro Lys Asp Ala Tyr Gly Ile Ser Gln Val Leu Trp Gly Ser Ile Gly
                   405                 410                 415
   Phe Thr Thr Gly Ala Thr Leu Gly Ala Ala Phe Ala Ala Glu Glu Ile
               420                 425                 430
   Asp Pro Asn Lys Arg Val Ile Leu Phe Ile Gly Asp Gly Ser Leu Gln
           435                 440                 445
   Leu Thr Val Gln Glu Ile Ser Thr Met Ile Arg Trp Gly Leu Lys Pro
       450                 455                 460
   Tyr Leu Phe Val Leu Asn Asn Asp Gly Tyr Thr Ile Glu Lys Leu Ile
   465                 470                 475                 480
   His Gly Pro His Ala Glu Tyr Asn Glu Ile Gln Thr Trp Asp His Leu
                   485                 490                 495
   Ala Leu Leu Pro Ala Phe Gly Ala Lys Lys Tyr Glu Asn His Lys Ile
               500                 505                 510
   Ala Thr Thr Gly Glu Trp Asp Ala Leu Thr Thr Asp Ser Glu Phe Gln
           515                 520                 525
   Lys Asn Ser Val Ile Arg Leu Ile Glu Leu Lys Leu Pro Val Phe Asp
   530 535 540
   Ala Pro Glu Ser Leu Ile Lys Gln Ala Gln Leu Thr Ala Ala Thr Asn
   545 550 555 560
   Ala Lys Gln
<210> 74
   <211> 506
   <212> PRT
   <213> Saccharomyces cerevisiae ALD2
<400> 74
   Met Pro Thr Leu Tyr Thr Asp Ile Glu Ile Pro Gln Leu Lys Ile Ser
   1 5 10 15
   Leu Lys Gln Pro Leu Gly Leu Phe Ile Asn Asn Glu Phe Cys Pro Ser
   20 25 30
   Ser Asp Gly Lys Thr Ile Glu Thr Val Asn Pro Ala Thr Gly Glu Pro
   35 40 45
   Ile Thr Ser Phe Gln Ala Ala Asn Glu Lys Asp Val Asp Lys Ala Val
   50 55 60
   Lys Ala Ala Arg Ala Ala Phe Asp Asn Val Trp Ser Lys Thr Ser Ser
   65 70 75 80
   Glu Gln Arg Gly Ile Tyr Leu Ser Asn Leu Leu Lys Leu Ile Glu Glu
   85 90 95
   Glu Gln Asp Thr Leu Ala Ala Leu Glu Thr Leu Asp Ala Gly Lys Pro
   100 105 110
   Tyr His Ser Asn Ala Lys Gly Asp Leu Ala Gln Ile Leu Gln Leu Thr
   115 120 125
   Arg Tyr Phe Ala Gly Ser Ala Asp Lys Phe Asp Lys Gly Ala Thr Ile
   130 135 140
   Pro Leu Thr Phe Asn Lys Phe Ala Tyr Thr Leu Lys Val Pro Phe Gly
   145 150 155 160
   Val Val Ala Gln Ile Val Pro Trp Asn Tyr Pro Leu Ala Met Ala Cys
   165 170 175
   Trp Lys Leu Gln Gly Ala Leu Ala Ala Gly Asn Thr Val Ile Ile Lys
   180 185 190
   Pro Ala Glu Asn Thr Ser Leu Ser Leu Leu Tyr Phe Ala Thr Leu Ile
   195 200 205
   Lys Lys Ala Gly Phe Pro Pro Gly Val Val Asn Ile Val Pro Gly Tyr
   210 215 220
   Gly Ser Leu Val Gly Gln Ala Leu Ala Ser His Met Asp Ile Asp Lys
   225 230 235 240
   Ile Ser Phe Thr Gly Ser Thr Lys Val Gly Gly Phe Val Leu Glu Ala
   245 250 255
   Ser Gly Gln Ser Asn Leu Lys Asp Val Thr Leu Glu Cys Gly Gly Lys
               260                 265                 270
   Ser Pro Ala Leu Val Phe Glu Asp Ala Asp Leu Asp Lys Ala Ile Asp
           275                 280                 285
   Trp Ile Ala Ala Gly Ile Phe Tyr Asn Ser Gly Gln Asn Cys Thr Ala
       290                 295                 300
   Asn Ser Arg Val Tyr Val Gln Ser Ser Ile Tyr Asp Lys Phe Val Glu
   305                 310                 315                 320
   Lys Phe Lys Glu Thr Ala Lys Lys Glu Trp Asp Val Ala Gly Lys Phe
                   325                 330                 335
   Asp Pro Phe Asp Glu Lys Cys Ile Val Gly Pro Val Ile Ser Ser Thr
               340                 345                 350
   Gln Tyr Asp Arg Ile Lys Ser Tyr Ile Glu Arg Gly Lys Arg Glu Glu
           355                 360                 365
   Lys Leu Asp Met Phe Gln Thr Ser Glu Phe Pro Ile Gly Gly Ala Lys
       370                 375                 380
   Gly Tyr Phe Ile Pro Pro Thr Ile Phe Thr Asp Val Pro Gln Thr Ser
   385                 390                 395                 400
   Lys Leu Leu Gln Asp Glu Ile Phe Gly Pro Val Val Val Val Ser Lys
                   405                 410                 415
   Phe Thr Asn Tyr Asp Asp Ala Leu Lys Leu Ala Asn Asp Thr Cys Tyr
               420                 425                 430
   Gly Leu Ala Ser Ala Val Phe Thr Lys Asp Val Lys Lys Ala His Met
           435                 440                 445
   Phe Ala Arg Asp Ile Lys Ala Gly Thr Val Trp Ile Asn Ser Ser Asn
       450                 455                 460
   Asp Glu Asp Val Thr Val Pro Phe Gly Gly Phe Lys Met Ser Gly Ile
   465                 470                 475                 480
   Gly Arg Glu Leu Gly Gln Ser Gly Val Asp Thr Tyr Leu Gln Thr Lys
                   485                 490                 495
   Ala Val His Ile Asn Leu Ser Leu Asp Asn
               500                 505
<210> 75
   <211> 506
   <212> PRT
   <213> Saccharomyces cerevisiae ALD3
<400> 75
   Met Pro Thr Leu Tyr Thr Asp Ile Glu Ile Pro Gln Leu Lys Ile Ser
   1               5                   10                  15
   Leu Lys Gln Pro Leu Gly Leu Phe Ile Asn Asn Glu Phe Cys Pro Ser
               20                  25                  30
   Ser Asp Gly Lys Thr Ile Glu Thr Val Asn Pro Ala Thr Gly Glu Pro
           35                  40                  45
   Ile Thr Ser Phe Gln Ala Ala Asn Glu Lys Asp Val Asp Lys Ala Val
       50                  55                  60
   Lys Ala Ala Arg Ala Ala Phe Asp Asn Val Trp Ser Lys Thr Ser Ser
   65                  70                  75                  80
   Glu Gln Arg Gly Ile Tyr Leu Ser Asn Leu Leu Lys Leu Ile Glu Glu
                   85                  90                  95
   Glu Gln Asp Thr Leu Ala Ala Leu Glu Thr Leu Asp Ala Gly Lys Pro
               100                 105                 110
   Phe His Ser Asn Ala Lys Gln Asp Leu Ala Gln Ile Ile Glu Leu Thr
           115                 120                 125
   Arg Tyr Tyr Ala Gly Ala Val Asp Lys Phe Asn Met Gly Glu Thr Ile
       130                 135                 140
   Pro Leu Thr Phe Asn Lys Phe Ala Tyr Thr Leu Lys Val Pro Phe Gly
   145                 150                 155                 160
   Val Val Ala Gln Ile Val Pro Trp Asn Tyr Pro Leu Ala Met Ala Cys
                   165                 170                 175
   Arg Lys Met Gln Gly Ala Leu Ala Ala Gly Asn Thr Val Ile Ile Lys
               180                 185                 190
   Pro Ala Glu Asn Thr Ser Leu Ser Leu Leu Tyr Phe Ala Thr Leu Ile
           195                 200                 205
   Lys Lys Ala Gly Phe Pro Pro Gly Val Val Asn Val Ile Pro Gly Tyr
       210                 215                 220
   Gly Ser Val Val Gly Lys Ala Leu Gly Thr His Met Asp Ile Asp Lys
   225                 230                 235                 240
   Ile Ser Phe Thr Gly Ser Thr Lys Val Gly Gly Ser Val Leu Glu Ala
                   245                 250                 255
   Ser Gly Gln Ser Asn Leu Lys Asp Ile Thr Leu Glu Cys Gly Gly Lys
               260                 265                 270
   Ser Pro Ala Leu Val Phe Glu Asp Ala Asp Leu Asp Lys Ala Ile Glu
           275                 280                 285
   Trp Val Ala Asn Gly Ile Phe Phe Asn Ser Gly Gln Ile Cys Thr Ala
       290                 295                 300
   Asn Ser Arg Val Tyr Val Gln Ser Ser Ile Tyr Asp Lys Phe Val Glu
   305                 310                 315                 320
   Lys Phe Lys Glu Thr Ala Lys Lys Glu Trp Asp Val Ala Gly Lys Phe
                   325                 330                 335
   Asp Pro Phe Asp Glu Lys Cys Ile Val Gly Pro Val Ile Ser Ser Thr
               340                 345                 350
   Gln Tyr Asp Arg Ile Lys Ser Tyr Ile Glu Arg Gly Lys Lys Glu Glu
           355                 360                 365
   Lys Leu Asp Met Phe Gln Thr Ser Glu Phe Pro Ile Gly Gly Ala Lys
       370                 375                 380
   Gly Tyr Phe Ile Pro Pro Thr Ile Phe Thr Asp Val Pro Glu Thr Ser
   385                 390                 395                 400
   Lys Leu Leu Arg Asp Glu Ile Phe Gly Pro Val Val Val Val Ser Lys
                   405                 410                 415
   Phe Thr Asn Tyr Asp Asp Ala Leu Lys Leu Ala Asn Asp Thr Cys Tyr
               420                 425                 430
   Gly Leu Ala Ser Ala Val Phe Thr Lys Asp Val Lys Lys Ala His Met
           435                 440                 445
   Phe Ala Arg Asp Ile Lys Ala Gly Thr Val Trp Ile Asn Gln Thr Asn
       450                 455                 460
   Gln Glu Glu Ala Lys Val Pro Phe Gly Gly Phe Lys Met Ser Gly Ile
   465                 470                 475                 480
   Gly Arg Glu Ser Gly Asp Thr Gly Val Asp Asn Tyr Leu Gln Ile Lys
                   485                 490                 495
   Ser Val His Val Asp Leu Ser Leu Asp Lys
               500                 505
<210> 76
   <211> 713
   <212> PRT
   <213> Saccharomyces cerevisiae ACS1
<400> 76
   Met Ser Pro Ser Ala Val Gln Ser Ser Lys Leu Glu Glu Gln Ser Ser
    1               5                  10                  15
   Glu Ile Asp Lys Leu Lys Ala Lys Met Ser Gln Ser Ala Ala Thr Ala
               20                  25                  30
   Gln Gln Lys Lys Glu His Glu Tyr Glu His Leu Thr Ser Val Lys Ile
           35                  40                  45
   Val Pro Gln Arg Pro Ile Ser Asp Arg Leu Gln Pro Ala Ile Ala Thr
       50                  55                  60
   His Tyr Ser Pro His Leu Asp Gly Leu Gln Asp Tyr Gln Arg Leu His
   65                  70                  75                  80
   Lys Glu Ser Ile Glu Asp Pro Ala Lys Phe Phe Gly Ser Lys Ala Thr
                   85                  90                  95
   Gln Phe Leu Asn Trp Ser Lys Pro Phe Asp Lys Val Phe Ile Pro Asp
               100                 105                 110
   Pro Lys Thr Gly Arg Pro Ser Phe Gln Asn Asn Ala Trp Phe Leu Asn
           115                 120                 125
   Gly Gln Leu Asn Ala Cys Tyr Asn Cys Val Asp Arg His Ala Leu Lys
       130                 135                 140
   Thr Pro Asn Lys Lys Ala Ile Ile Phe Glu Gly Asp Glu Pro Gly Gln
   145                 150                 155                 160
   Gly Tyr Ser Ile Thr Tyr Lys Glu Leu Leu Glu Glu Val Cys Gln Val
   165 170 175
   Ala Gln Val Leu Thr Tyr Ser Met Gly Val Arg Lys Gly Asp Thr Val
   180 185 190
   Ala Val Tyr Met Pro Met Val Pro Glu Ala Ile Ile Thr Leu Leu Ala
   195 200 205
   Ile Ser Arg Ile Gly Ala Ile His Ser Val Val Phe Ala Gly Phe Ser
   210 215 220
   Ser Asn Ser Leu Arg Asp Arg Ile Asn Asp Gly Asp Ser Lys Val Val
   225 230 235 240
   Ile Thr Thr Asp Glu Ser Asn Arg Gly Gly Lys Val Ile Glu Thr Lys
   245 250 255
   Arg Ile Val Asp Asp Ala Leu Arg Glu Thr Pro Gly Val Arg His Val
   260 265 270
   Leu Val Tyr Arg Lys Thr Asn Asn Pro Ser Val Ala Phe His Ala Pro
   275 280 285
   Arg Asp Leu Asp Trp Ala Thr Glu Lys Lys Lys Tyr Lys Thr Tyr Tyr
   290 295 300
   Pro Cys Thr Pro Val Asp Ser Glu Asp Pro Leu Phe Leu Leu Tyr Thr
   305 310 315 320
   Ser Gly Ser Thr Gly Ala Pro Lys Gly Val Gln His Ser Thr Ala Gly
   325 330 335
   Tyr Leu Leu Gly Ala Leu Leu Thr Met Arg Tyr Thr Phe Asp Thr His
   340 345 350
   Gln Glu Asp Val Phe Phe Thr Ala Gly Asp Ile Gly Trp Ile Thr Gly
   355 360 365
   His Thr Tyr Val Val Tyr Gly Pro Leu Leu Tyr Gly Cys Ala Thr Leu
   370 375 380
   Val Phe Glu Gly Thr Pro Ala Tyr Pro Asn Tyr Ser Arg Tyr Trp Asp
   385 390 395 400
   Ile Ile Asp Glu His Lys Val Thr Gln Phe Tyr Val Ala Pro Thr Ala
   405 410 415
   Leu Arg Leu Leu Lys Arg Ala Gly Asp Ser Tyr Ile Glu Asn His Ser
   420 425 430
   Leu Lys Ser Leu Arg Cys Leu Gly Ser Val Gly Glu Pro Ile Ala Ala
   435 440 445
   Glu Val Trp Glu Trp Tyr Ser Glu Lys Ile Gly Lys Asn Glu Ile Pro
   450 455 460
   Ile Val Asp Thr Tyr Trp Gln Thr Glu Ser Gly Ser His Leu Val Thr
   465 470 475 480
   Pro Leu Ala Gly Gly Val Thr Pro Met Lys Pro Gly Ser Ala Ser Phe
   485 490 495
   Pro Phe Phe Gly Ile Asp Ala Val Val Leu Asp Pro Asn Thr Gly Glu
   500 505 510
   Glu Leu Asn Thr Ser His Ala Glu Gly Val Leu Ala Val Lys Ala Ala
   515 520 525
   Trp Pro Ser Phe Ala Arg Thr Ile Trp Lys Asn His Asp Arg Tyr Leu
   530 535 540
   Asp Thr Tyr Leu Asn Pro Tyr Pro Gly Tyr Tyr Phe Thr Gly Asp Gly
   545 550 555 560
   Ala Ala Lys Asp Lys Asp Gly Tyr Ile Trp Ile Leu Gly Arg Val Asp
   565 570 575
   Asp Val Val Asn Val Ser Gly His Arg Leu Ser Thr Ala Glu Ile Glu
   580 585 590
   Ala Ala Ile Ile Glu Asp Pro Ile Val Ala Glu Cys Ala Val Val Gly
   595 600 605
   Phe Asn Asp Asp Leu Thr Gly Gln Ala Val Ala Ala Phe Val Val Leu
   610 615 620
   Lys Asn Lys Ser Ser Trp Ser Thr Ala Thr Asp Asp Glu Leu Gln Asp
   625 630 635 640
   Ile Lys Lys His Leu Val Phe Thr Val Arg Lys Asp Ile Gly Pro Phe
   645 650 655
   Ala Ala Pro Lys Leu Ile Ile Leu Val Asp Asp Leu Pro Lys Thr Arg
   660 665 670
   Ser Gly Lys Ile Met Arg Arg Ile Leu Arg Lys Ile Leu Ala Gly Glu
   675 680 685
   Ser Asp Gln Leu Gly Asp Val Ser Thr Leu Ser Asn Pro Gly Ile Val
   690 695 700
   Arg His Leu Ile Asp Ser Val Lys Leu
   705 710
<210> 77
   <211> 348
   <212> PRT
   <213> Saccharomyces cerevisiae ADH1
<400> 77
   Met Ser Ile Pro Glu Thr Gln Lys Gly Val Ile Phe Tyr Glu Ser His
    1               5                  10                  15
   Gly Lys Leu Glu Tyr Lys Asp Ile Pro Val Pro Lys Pro Lys Ala Asn
               20                  25                  30
   Glu Leu Leu Ile Asn Val Lys Tyr Ser Gly Val Cys His Thr Asp Leu
           35                  40                  45
   His Ala Trp His Gly Asp Trp Pro Leu Pro Val Lys Leu Pro Leu Val
       50                  55                  60
   Gly Gly His Glu Gly Ala Gly Val Val Val Gly Met Gly Glu Asn Val
   65                  70                  75                  80
   Lys Gly Trp Lys Ile Gly Asp Tyr Ala Gly Ile Lys Trp Leu Asn Gly
   85 90 95
   Ser Cys Met Ala Cys Glu Tyr Cys Glu Leu Gly Asn Glu Ser Asn Cys
   100 105 110
   Pro His Ala Asp Leu Ser Gly Tyr Thr His Asp Gly Ser Phe Gln Gln
   115 120 125
   Tyr Ala Thr Ala Asp Ala Val Gln Ala Ala His Ile Pro Gln Gly Thr
   130 135 140
   Asp Leu Ala Gln Val Ala Pro Ile Leu Cys Ala Gly Ile Thr Val Tyr
   145 150 155 160
   Lys Ala Leu Lys Ser Ala Asn Leu Met Ala Gly His Trp Val Ala Ile
   165 170 175
   Ser Gly Ala Ala Gly Gly Leu Gly Ser Leu Ala Val Gln Tyr Ala Lys
   180 185 190
   Ala Met Gly Tyr Arg Val Leu Gly Ile Asp Gly Gly Glu Gly Lys Glu
   195 200 205
   Glu Leu Phe Arg Ser Ile Gly Gly Glu Val Phe Ile Asp Phe Thr Lys
   210 215 220
   Glu Lys Asp Ile Val Gly Ala Val Leu Lys Ala Thr Asp Gly Gly Ala
   225 230 235 240
   His Gly Val Ile Asn Val Ser Val Ser Glu Ala Ala Ile Glu Ala Ser
   245 250 255
   Thr Arg Tyr Val Arg Ala Asn Gly Thr Thr Val Leu Val Gly Met Pro
   260 265 270
   Ala Gly Ala Lys Cys Cys Ser Asp Val Phe Asn Gln Val Val Lys Ser
   275 280 285
   Ile Ser Ile Val Gly Ser Tyr Val Gly Asn Arg Ala Asp Thr Arg Glu
   290 295 300
   Ala Leu Asp Phe Phe Ala Arg Gly Leu Val Lys Ser Pro Ile Lys Val
   305 310 315 320
   Val Gly Leu Ser Thr Leu Pro Glu Ile Tyr Glu Lys Met Glu Lys Gly
   325 330 335
   Gln Ile Val Gly Arg Tyr Val Val Asp Thr Ser Lys
   340 345
<210> 78
   <211> 587
   <212> PRT
   <213> Saccharomyces cerevisiae DLD1
<400> 78
   Met Leu Trp Lys Arg Thr Cys Thr Arg Leu Ile Lys Pro Ile Ala Gln
    1               5                  10                  15
   Pro Arg Gly Arg Leu Val Arg Arg Ser Cys Tyr Arg Tyr Ala Ser Thr
               20                  25                  30
   Gly Thr Gly Ser Thr Asp Ser Ser Ser Gln Trp Leu Lys Tyr Ser Val
   35 40 45
   Ile Ala Ser Ser Ala Thr Leu Phe Gly Tyr Leu Phe Ala Lys Asn Leu
   50 55 60
   Tyr Ser Arg Glu Thr Lys Glu Asp Leu Ile Glu Lys Leu Glu Met Val
   65 70 75 80
   Lys Lys Ile Asp Pro Val Asn Ser Thr Leu Lys Leu Ser Ser Leu Asp
   85 90 95
   Ser Pro Asp Tyr Leu His Asp Pro Val Lys Ile Asp Lys Val Val Glu
   100 105 110
   Asp Leu Lys Gln Val Leu Gly Asn Lys Pro Glu Asn Tyr Ser Asp Ala
   115 120 125
   Lys Ser Asp Leu Asp Ala His Ser Asp Thr Tyr Phe Asn Thr His His
   130 135 140
   Pro Ser Pro Glu Gln Arg Pro Arg Ile Ile Leu Phe Pro His Thr Thr
   145 150 155 160
   Glu Glu Val Ser Lys Ile Leu Lys Ile Cys His Asp Asn Asn Met Pro
   165 170 175
   Val Val Pro Phe Ser Gly Gly Thr Ser Leu Glu Gly His Phe Leu Pro
   180 185 190
   Thr Arg Ile Gly Asp Thr Ile Thr Val Asp Leu Ser Lys Phe Met Asn
   195 200 205
   Asn Val Val Lys Phe Asp Lys Leu Asp Leu Asp Ile Thr Val Gln Ala
   210 215 220
   Gly Leu Pro Trp Glu Asp Leu Asn Asp Tyr Leu Ser Asp His Gly Leu
   225 230 235 240
   Met Phe Gly Cys Asp Pro Gly Pro Gly Ala Gln Ile Gly Gly Cys Ile
   245 250 255
   Ala Asn Ser Cys Ser Gly Thr Asn Ala Tyr Arg Tyr Gly Thr Met Lys
   260 265 270
   Glu Asn Ile Ile Asn Met Thr Ile Val Leu Pro Asp Gly Thr Ile Val
   275 280 285
   Lys Thr Lys Lys Arg Pro Arg Lys Ser Ser Ala Gly Tyr Asn Leu Asn
   290 295 300
   Gly Leu Phe Val Gly Ser Glu Gly Thr Leu Gly Ile Val Thr Glu Ala
   305 310 315 320
   Thr Val Lys Cys His Val Lys Pro Lys Ala Glu Thr Val Ala Val Val
   325 330 335
   Ser Phe Asp Thr Ile Lys Asp Ala Ala Ala Cys Ala Ser Asn Leu Thr
   340 345 350
   Gln Ser Gly Ile His Leu Asn Ala Met Glu Leu Leu Asp Glu Asn Met
   355 360 365
   Met Lys Leu Ile Asn Ala Ser Glu Ser Thr Asp Arg Cys Asp Trp Val
       370                 375                 380
   Glu Lys Pro Thr Met Phe Phe Lys Ile Gly Gly Arg Ser Pro Asn Ile
   385                 390                 395                 400
   Val Asn Ala Leu Val Asp Glu Val Lys Ala Val Ala Gln Leu Asn His
                   405                 410                 415
   Cys Asn Ser Phe Gln Phe Ala Lys Asp Asp Asp Glu Lys Leu Glu Leu
               420                 425                 430
   Trp Glu Ala Arg Lys Val Ala Leu Trp Ser Val Leu Asp Ala Asp Lys
           435                 440                 445
   Ser Lys Asp Lys Ser Ala Lys Ile Trp Thr Thr Asp Val Ala Val Pro
       450                 455                 460
   Val Ser Gln Phe Asp Lys Val Ile His Glu Thr Lys Lys Asp Met Gln
   465                 470                 475                 480
   Ala Ser Lys Leu Ile Asn Ala Ile Val Gly His Ala Gly Asp Gly Asn
                   485                 490                 495
   Phe His Ala Phe Ile Val Tyr Arg Thr Pro Glu Glu His Glu Thr Cys
               500                 505                 510
   Ser Gln Leu Val Asp Arg Met Val Lys Arg Ala Leu Asn Ala Glu Gly
           515                 520                 525
   Thr Cys Thr Gly Glu His Gly Val Gly Ile Gly Lys Arg Glu Tyr Leu
       530                 535                 540
   Leu Glu Glu Leu Gly Glu Ala Pro Val Asp Leu Met Arg Lys Ile Lys
   545                 550                 555                 560
   Leu Ala Ile Asp Pro Lys Arg Ile Met Asn Pro Asp Lys Ile Phe Lys
                   565                 570                 575
   Thr Asp Pro Asn Glu Pro Ala Asn Asp Tyr Arg
               580                 585

## Description

### [Technical Field]

The present invention relates to a lactic acid-producing isolated *Saccharomyces cerevisiae* microorganism, and a method for producing lactic acid from the culture medium containing the microorganism by culturing the same.

### [Background Art]

Generally, lactic acid is an important organic acid with a wide range of applications including food additives such as food preservative, fragrance, or acidifier etc., and has been used broadly for industrial purposes such as cosmetics, chemistry, metals, electronics, fabrics, dyeing textiles, and pharmaceutical industries, etc. In addition, lactic acid is an essential ingredient of polylactic acid, one of biodegradable plastics, and thus the demand for lactic acid has been increasing significantly. It is also used as an important material for the productions of many chemical compounds including polylactic acid, acetaldehyde, polypropylene glycol, acrylic acid, 2,3-pentathione, etc. Specifically, D-type lactic acid is an essential ingredient for producing stereocomplex PLA, which is an optical isomer required for the production of highly heat-resistant PLA.

Specifically, the method for producing lactic acid includes a traditional chemical synthesis and a biological fermentation process. When lactic acid is produced via the chemical synthesis, lactic acid is produced in the form of a racemic mixture consisting of 50% D-type lactic acid and 50% L-type lactic acid, and it is difficult to control the composition ratio, and thus polylactic acid produced therefrom may become an amorphous polymer having a low melting point, thereby imposing limitations on the development of their use. On the other hand, the biological fermentation process allows to selectively produce D-type lactic acid or L-type lactic acid depending on the strain used. Thus, the latter is preferred commercially because it is possible to produce a particular isoform of lactic acid.

Meanwhile, attempts have been made in order to improve the productivity of lactic acid via various gene manipulations using a *saccharomyces sp.* microorganism having D-lactic acid-producing ability, by introducing a gene of an enzyme for conversion into D-type lactic acid. Specifically, attempts have been made to improve the productivity of lactic acid by strengthening the activity of lactic acid dehydrogenase (LDH) while decreasing the activities of pyruvate decarboxylase (PDC), aldehyde dehydrogenase (ALD), and/or acetyl-CoA synthetase (ACS), and (U.S. Patent Application Publication NOs. 2012-021421, 2010-0248233, and 2006-0148050). However, the overall fermentation productivity was low due to the low cell growth of the lactic acid-producing strain.

Accordingly, intensive efforts have been made by the present inventors in order to obtain a microorganism having enhanced lactic acid productivity with an efficient cell growth while the activities of PDC isotypes were controlled. As a result, it has been confirmed that strains, in which the activity of PDC1 is inactivated and the activity of PDC5 is decreased, or the activity of PDC1 is decreased and the activity of PDC5 is inactivated and the activities of aldehyde dehydrogenase (ALD) and acetyl-CoA synthetase (ACS) of the microorganism were enhanced, were able to increase the lactic acid production yield and facilitate the cell growth of the strains, thereby improving the overall lactic acid fermentation productivity, and this has led to the completion of the present invention.

### [Disclosure]

### [Technical Problem]

An objective of the present invention is to provide a *Saccharomyces cerevisiae* microorganism having enhanced productivity of lactic acid.

Another objective of the present invention is to provide a method for producing lactic acid using the *Saccharomyces cerevisiae* microorganism.

### [Technical Solution]

In a first aspect of the present invention, to achieve the objectives described above, there is provided an isolated *Saccharomyces cerevisiae* microorganism having enhanced productivity of lactic acid, wherein the microorganism is modified so that: (a) (i) the activity of PDC1 is inactivated and the activity of PDC5 is decreased, or (ii) the activity of PDC1 is decreased and the activity of PDC5 is inactivated; and (b) the activities of aldehyde dehydrogenase (ALD) and acetyl-CoA synthetase (ACS) are enhanced, wherein the activity is enhanced by an insertion of a plasmid containing the genes of an enzyme, an increase in the number of gene copies encoding an enzyme on a chromosome, or an increase in an enzyme activity caused by a substitution or modification, or a mutation of a promoter sequence of an enzyme gene; wherein the activity is decreased by substitution or modification of a promoter sequence of the enzyme gene, or mutation into an enzyme having decreased activity caused by a substitution or modification of the enzyme; and wherein the activity is inactivated by a partial gene deletion or a whole gene deletion, an insertion of a foreign-derived gene into the relevant gene, substitution or modification of a gene promoter sequence of the enzyme, or a mutation into an inactive-enzyme having a loss in its original functions caused by a substitution or modification of the enzyme.

Generally, a lactic acid-producing *Saccharomyces sp.* microorganism produces lactic acid via lactic acid dehydrogenase (LDH) using pyruvate as a substrate. Ethanol fermentation pathway and acetyl-CoA production pathway, the representative metabolic pathways utilizing pyruvate as a common substrate, were blocked. Decreasing PDC activity may be helpful in the production of lactic acid and the yield improvement thereof, however, when the level of decrease reached a certain level, insufficient amount of cytosolic acetyl-CoA was produced, which in turn, blocked the cell growth, and thus the normal fermentation was not achieved. Accordingly, the present inventor developed a *Saccharomyces cerevisiae* microorganism having enhanced productivity of lactic acid by improving the overall lactic acid fermentation productivity, in which the growth rate of the microorganism was maintained with improved lactic acid productivity yield by regulating the acetyl-CoA pathway at a minimum level.

The term "pyruvate decarboxylase (PDC)" used herein refers to a protein having an activity capable of mediating a reaction responsible for producing carbonic acid and acetaldehyde from pyruvate. The protein has been known to be involved in a step of alcohol fermentation, and is mostly present in yeasts and plants. The pyruvate decarboxylase of the present invention is PDC1 andPDC5 of *Saccharomyces cerevisiae.* The protein may include any variants or analogues thereof as long as they are biologically identical and have corresponding activities to the protein. The amino acid sequences of the protein may be obtained from a known database, etc., e.g., GenBank of NCBI, etc.. Specifically, PDC1 may consist of an amino acid sequence of SEQ ID NO: 71, PDC5 of an amino acid sequence of SEQ ID NO: 72. Further is described herein PDC 6 of an amino acid sequence of SEQ ID NO: 73. The protein may include amino acid sequences having a homology of more than 70%, specifically more than 80%, more specifically more than 90%, and even more specifically more than 95%, to each of the above-listed amino acid sequences. Any variant of the above-listed sequences encoding the same amino acid sequences, which results from genetic code degeneracy, may also be included in the present invention.

The term "homology" used herein refers to a degree of similarity between a plurality of nucleotide sequences or amino acid sequences, and is a unit representing a sequence having the same sequences to the amino acid sequences or the nucleotide sequences of the present invention, with a probability equal to or greater than the above probability. Such homology may be determined by comparing the two given sequences with the naked eye, but rather, it may be measured using a sequence comparison program, which is easily accessible, that interprets the degree of homology by arranging the sequences to be compared side by side. The sequence comparison programs known in the art include FASTP, BLAST, BLAST2, PSIBLAST, and a software containing CLUSTAL W, etc.

Numerous examples have been reported regarding the production of lactic acid by allowing a defect in PDC1, whose exhibits a major activity in lactic acid production (Appl. Microbiol. Biotechnol. 2009, 82(5):883-90). In this case, since PDC6 is rarely expressed, the actual PDC activity as appears to be due to the expression of PDC5 gene. According to the reports, the defect in PDC1 alone does not hinder the cell growth of a wild-type strain, and also about 60-70% of PDC activity can be maintained compared to that of the wild-type, and thus no significant phenotypic change has been observed in the strain (J. Bacteriol. 1990, 172(2):678-685).

As an alternative, a strain having a simultaneous double defect in both PDC1 and PDC5 genes, which exhibit major PDC activities in yeasts, may be prepared. In such case, lactic acid fermentation can be carried out using a sugar source such as glycogen in the absence of a co-substrate such as acetic acid or ethanol. However, it resulted in decrease in the growth rate of the yeast strain due to a rapid decrease in PDC activity, thereby reducing the fermentation productivity of lactic acid (Biosci. Biotechnol. Biochem. 2006, 70(5):1148-1153).

Meanwhile, in order to maximize the lactic acid via LDH pathway, which competes with PDC for pyruvate, a strain with a simultaneous triple defect in PDC1, PDC5, and PDC6 may be prepared. In this case, lactic acid fermentation yield may be maximized but the metabolic capabilities of ethanol and acetic acid due to catabolite repression induced in the presence of glucose may be further inhibited, thereby reducing cell growth and ultimately leading to decrease in fermentation productivity (Curr. Genet. 2003, 43(3): 139-160).

According to the present invention, the decrease in pyruvate decarboxylase (PDC) activity of the present invention is to i) inactivate PDC1 activity and decrease PDC5 activity; or to ii) decrease PDC1 activity and inactivate PDC5 activity.

In an exemplary embodiment of the present invention, four different strains, which include a strain with a decreased PDC5 activity by substituting the promoter of PDC5 gene, a strain that caused a defect in PDC5 gene by recovering PDC1 activity, a strain that caused a double defect in PDC1 and PDC5 genes, and a strain that caused a triple defect in PDC1, PDC5, and PDC6 genes, were prepared based on *a Saccharomyces cerevisiae* strain in which PDC1 activity was inactivated. Among the thus prepared strains, the strain having a triple gene defect was shown to rarely undergo cell growth.

The term "aldehyde dehydrogenase (ALD)" used herein refers to a protein having an activity of mainly producing acetic acid from acetaldehyde as a protein having an activity of producing carboxylic acid or an acyl group by the oxidation of aldehyde in the present invention. The aldehyde dehydrogenase of the present invention is derived from a *Saccharomyces cerevisiae* microorganism, or may be ALD2 and/or ALD3. Specifically, the protein may be ALD2 and/or ALD3 of *Saccharomyces cerevisiae*, and may include any variant or an analogue thereof as long as they are biologically identical and have corresponding activities to the protein. The amino acid sequences of the protein may be obtained from database, etc., known in the art, e.g., GenBank of NCBI, etc.. Specifically, ALD2 may consist of an amino acid sequence of SEQ ID NO: 74, and ALD3 may consist of an amino acid sequence of SEQ ID NO: 75. The protein may include amino acid sequences having a homology of more than 70%, specifically more than 80%, more specifically more than 90%, and even more specifically more than 95%, to the amino acid sequences. Any variant of the sequences encoding the identical amino acid sequences, which results from genetic code degeneracy, may also be included in the present invention.

The term "acetyl-CoA synthetase (ACS)" used herein refers to a protein having an activity of catalyzing the thioesterification of acetic acid and CoA in conjugation with an ATP decomposition reaction. It has been known that the protein is present in microorganisms, plants, and animals, etc. The acetyl-CoA synthetase of the present invention may be derived from a *Saccharomyces sp.* microorganism, or may be ACS1. Specifically, the protein may be ACS1 of *Saccharomyces cerevisiae*, and may include any variant or an analogue thereof as long as they are biologically identical and have corresponding activities to the protein. The amino acid sequences of the protein may be obtained from a known database, etc., e.g., GenBank of NCBI, etc.. Specifically, ACS1 may be composed of an amino acid sequence of SEQ ID NO: 76, and may include amino acid sequences having a homology of more than 70%, specifically more than 80%, more specifically more than 90%, and even more specifically more than 95%, to the amino acid sequence. A protein mutant of the sequence encoding the identical amino acid sequences, which results from genetic code degeneracy, may also be included in the present invention.

In an exemplary embodiment of the present invention, strains, in which the activities of ALD2 and ACS, or the activities of ALD3 and ACS were increased, were prepared based on the strain having a decreased PDC activity compared to that of a non-mutated microorganism. According to the present invention, strains having increased activities of ALD and ACS were prepared based on the strain having inactivated PDC1 via PDC1 defect and decreased PDC5 activity by substituting the gene promoter of PDC5 with a promoter having low expression ability. More specifically, according to the present invention, strains of *Saccharomyces cerevisiae* microorganism, in which PDC1 activity was inactivated, PDC5 activity was decreased, the activity of at least one selected from the group consisting of ALD2 and ALD3 was increased, and ACS1 activity was increased, were prepared. Accordingly, it was confirmed that the growth rate of the strains, D-lactic acid production rate and the yield thereof were significantly improved.

The term "inactivation" of an enzyme activity of the present invention refers to a method for inactivating enzyme activities including any method that inhibits the expression of an enzyme, or allows the expression of an enzyme incapable of exhibiting its original activities. The method may include a partial gene deletion or a whole gene deletion caused by a homology recombination, an inhibition of an enzyme expression caused by an insertion of a foreign-derived gene into the relevant gene, an inhibition of an enzyme expression caused by a substitution or modification of a gene promoter sequence of the enzyme, or a mutation into an inactive-enzyme having a loss in its original functions caused by a substitution or modification of the enzyme, etc..

The term "decrease" of an enzyme activity used herein refers to a method for decreasing the activity of an enzyme including any method for decreasing the expression level of an enzyme, or decreasing the activity of an enzyme being expressed. The method may include a decrease in an expression caused by a substitution or modification of a promoter sequence of the enzyme gene, or a mutation into an enzyme having decreased activity caused by a substitution or modification of the enzyme, etc..

The term "increase" of an enzyme activity used herein refers to an insertion of a plasmid containing the genes of an enzyme, an increase in the number of gene copies encoding an enzyme on a chromosome, or an increase in an enzyme activity caused by a substitution or modification, or a mutation of a promoter sequence of an enzyme gene, etc..

The term "yeast microorganism" used herein refers to a microorganism belonging to *Eumycetes* that proliferates by germination, as long as it is involved in any one of the lactic acid production pathway, alcohol production pathway, and/or acetyl-CoA production pathway. The yeast microorganism may be classified into *Saccharomyces sp., Pichia sp., Candida sp.,* and *Saccharomycopsis sp.,* depending on the shape of the yeast, and specifically, *saccharomyces sp.,* which includes various species. It is described herein that the microorganism may be selected from the group consisting of *Saccharomyces bayanus, Saccharomyces boulardii, Saccharomyces bulderi, Saccharomyces cariocanus, Saccharomyces cariocus, Saccharomyces cerevisiae*, *Saccharomyces chevalieri, Saccharomyces dairenensis, Saccharomyces ellipsoideus, Saccharomyces eubayanus, Saccharomyces exiguus, Saccharomyces florentinus, Saccharomyces kluyveri, Saccharomyces martiniae, Saccharomyces monacensis, Saccharomyces norbensis, Saccharomyces paradoxus, Saccharomyces pastorianus, Saccharomyces spencerorum, Saccharomyces turicensis, Saccharomyces unisporus, Saccharomyces uvarum,* and *Saccharomyces zonatus.* The microorganism applied in the present invention is *Saccharomyces cerevisiae.*

By preparing this microorganism having an i) inactivated PDC1 activity and a decreased PDC5 activity; or ii) decreased PDC1 activity and inactivated PDC5 activity and enhanced activities of ALD and ACS based on *Saccharomyces cerevisiae,* a representative example of *Saccharomyces sp.,* a significant increase in lactic acid production was confirmed.

The microorganism of the present invention may include alcohol dehydrogenase (ADH) that is to be further inactivated.

The term "alcohol dehydrogenase" used herein refers to a protein having an activity of catalyzing a reverse reaction responsible for producing aldehyde or ketone by removing hydrogen from alcohol. Alcohol dehydrogenase of the present invention may be derived from *Saccharomyces sp*., or may be ADH1. Specifically, the protein may be ADH1 of *Saccharomyces cerevisiae*, and may include any variant or an analogue thereof as long as they are biologically identical and have corresponding activities to the protein. The amino acid sequences of the protein may be obtained from a known database etc., e.g., GenBank of NCBI, etc.. Specifically, ADH1 may be composed of an amino acid sequence of SEQ ID NO: 77, and may include amino acid sequences having a homology of more than 70%, specifically more than 80%, more specifically more than 90%, and even more specifically more than 95%, to the amino acid sequence. A protein mutant of the sequence encoding the identical amino acid sequences, which results from genetic code degeneracy, may also be included in the present invention.

The microorganism of the present invention may include D-lactic acid dehyrogenase (DLD) that is further inactivated.

The term "D-lactic acid dehydrogenase" used herein, refers to a protein having an activity of producing pyruvate by anhydrization of D-lactic acid. D-lactic acid dehydrogenase of the present invention may be derived from *Saccharomyces sp..* Specifically, the protein may be DLD1 of *Saccharomyces cerevisiae,* and may include any variant or an analogue thereof as long as they are biologically identical and have corresponding activities to the protein. The amino acid sequences of the protein may be obtained from a known database, etc., e.g., GenBank of NCBI. Specifically, DLD1 may consist of an amino acid sequence of SEQ ID NO: 78, and may include amino acid sequences having a homology of more than 70%, specifically more than 80%, more specifically more than 90%, and even more specifically more than 95%, to the amino acid sequence. Any variant of the sequence encoding the identical amino acid sequences, which results from genetic code degeneracy, may also be included in the present invention.

In the present invention, the strains having a defect in ADH1, an enzyme involved in alcohol fermentation pathway using aldehyde as a substrate, which is further produced from pyruvate, and a defect in DLD1, an enzyme that decomposes the produced lactic acid, were used to precisely measure the changes in the lactic acid fermentation cell performances according to the regulation of acetic acid production pathway. In an exemplary embodiment of the present invention, the strains, in which the activities of PDC, ALD, and ACS were regulated, showed a significant increase in the lactic acid fermentation productivity. The results are summarized in Table 12.

In another aspect, the present invention provides a method for producing lactic acid using the microorganism of the present invention.

Specifically, in an exemplary embodiment of the present invention, the present invention provides a method for producing lactic acid including a) culturing the microorganism of the present invention in the culture medium and b) recovering lactic acid from the culture medium or the microorganism in step a).

The culturing may be performed using an appropriate medium and culturing conditions known in the art. According to the strains used, the culturing process may be readily adjusted by one of ordinary skill in the art. Examples of culturing methods include batch type, continuous type, and fed-batch type. The media used in the culturing process should appropriately meet the requirements of a specific strain.

The medium used in the present invention contains sucrose or glucose as a main carbon source, and molasses containing a high concentration of sucrose may also be used as a carbon source. Other carbon sources may be used in an adequate amount variously. Organic nitrogen sources including peptone, yeast extract, meat extract, malt extract, corn steep liquor, and soybean wheat, and inorganic nitrogen sources including element, ammonium sulfate, ammonium chloride, ammonium phosphate, ammonium carbonate, and ammonium nitrate may be used as a nitrogen source. These nitrogen sources may be employed either singly or in combination. To the medium, phosphorus sources such as potassium dihydrogen phosphate, dipotassium hydrogen phosphate, or corresponding sodium-containing salts may be added. In addition, the medium may contain metal salts such as magnesium sulfate and iron sulfate. Further, the medium may be supplemented with amino acids, vitamins, and appropriate precursors. These media or precursors may be added to cultures by a batch type or continuous type method.

During the culturing process, compounds such as ammonium hydroxide, potassium hydroxide, phosphoric acid, and sulfuric acid may be properly added in order to adjust the pH of the culture. Further, a defoaming agent such as fatty acid polyglycol ester may be added in order to inhibit the formation of foams in the culture. In addition, to maintain the culture in an aerobic condition, oxygen or oxygen-containing gas may be injected into the culture, and to maintain the culture in anaerobic and micro-aerobic conditions, nitrogen, hydrogen, or carbon dioxide gases may be injected into the culture without injecting any gas.

The temperature of the culture may be maintained at 20 to 40°C, specifically at 25 to 35°C, and more specifically at 30°C. The culturing may be continued until a desired amount of the desired material is obtained, and specifically for 10 to 100 hours.

The lactic acid produced in the culturing processes of the present invention may be collected from the culture medium by a proper method known in the art, depending on the culturing method, e.g., batch type, continuous type, or fed-batch type.

### [Advantageous Effects]

The present invention relates to using an isolated *Saccharomyces cerevisiae* microorganism having enhanced lactic acid fermentation productivity by controlling the activities of PDC isotypes, and enhancing the activities of aldehyde dehydrogenase (ALD) and acetyl-CoA synthetase (ACS). Therefore, it can be extensively used in the lactic acid fermentation production industries.

### [Brief Description of Drawings]

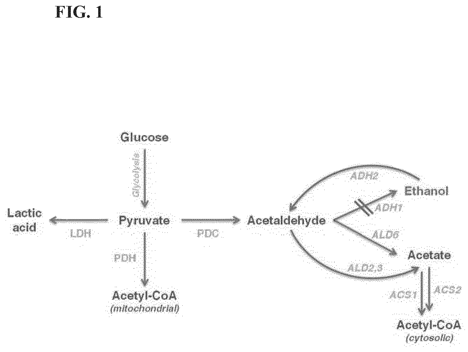

FIG.1 is a schematic diagram illustrating the relationship between the lactic acid production pathway of a *Saccharomyces sp.* microorganism, the alcohol fermentation pathway and the acetyl-CoA production pathway.

### [Best Mode for Carrying Out Invention]

Herein below, the present invention will be described in detail with accompanying exemplary embodiments. However, the exemplary embodiments disclosed herein are only for illustrative purposes and should not be construed as limiting the scope of the present invention.

### Example 1: Preparation of lactic acid-producing strain

To prepare lactic acid-producing strains, *Saccharomyces cerevisiae* CEN.PK2-1D, a representative wild type yeast obtained from EUROSCARF, was subject to genetic manipulation.

Specifically, a strain, where alcohol dehydrogenase 1 (ADH1) and pyruvate decarboxylase 1 (PDC1) were defective to minimize the loss of pyruvate to the alcohol synthesis pathway, and D-lactic acid dehydrogenase 1 (DLD1) was defective for blocking the D-type lactic acid decomposition pathway, was used as a base strain.

DLD1 is not a crucial factor that may have a direct impact on the growth improvement, but has been known as a major enzyme capable of converting D-lactic acid to pyruvate using NAD⁺ as D-lactic acid dehydrogenase. Accordingly, a subsequent strain was constructed based on the strain having gene defects in DLD1, an enzyme that consumes the prepared lactic acid thereof, to compare a complete fermentation productivity of D-type lactic acid-producing yeast, which is intended to be prepared in the present invention. As a result, the fermentation productivity was compared.

In the present invention, a general molecular cloning was employed for the gene manipulation.

First, in order to delete ADH1 and PDC1 genes of the yeast strains, an experiment was conducted with reference to the content disclosed in the Reference by Lee TH, et al. (J. Microbiol. Biotechnol. (2006), 16(6), 979-982), using plasmids pWAL100 and pWBR100. Each insert, which was introduced into the vector plasmids, was prepared using the suitable primers (corresponding to nucleotide sequences of SEQ ID NOs: 1 to 8) via PCR.

In addition, for the deletion of DLD1 gene, HIS3, which is a marker gene, was introduced by double crossover, and made it defective. The DNA fragments used therein were prepared using primers corresponding to nucleotide sequences of SEQ ID NOs: 9 and 10. The primers used in the gene manipulation are summarized in Table 1 below.

**Table 1**

| Primers used for the production of the base yeast strain | |
|---|---|
| Primer | 5'-> 3' sequence |
| ADH1 upstream forward primer (SEQ ID NO: 1) | CGGGATCCACTGTAGCCCTAGACTTGATAGCC |
| ADH1 upstream reverse primer (SEQ ID NO: 2) | |
| ADH1 downstream forward primer (SEQ ID NO: 3) | GACTAGTGCGAATTTCTTATGATTTATGATTTTTATT |
| ADH1 downstream reverse primer (SEQ ID NO: 4) | ACATGCCATGGAAGCATGCACGTATACACTTGAGTAA |
| PDC1 upstream forward primer (SEQ ID NO: 5) | CGGGATCCATTATGTATGCTCTTCTGACTTTTCGT |
| PDC1 upstream reverse primer (SEQ ID NO: 6) | |
| PDC1 downstream forward primer (SEQ ID NO: 7) | CGGGATCCGCGATTTAATCTCTAATTATTAGTTAAAG |
| PDC1 downstream reverse primer (SEQ ID NO: 8) | |
| DLD1-HIS3 upstream linking primer (SEQ ID NO: 9) | |
| DLD1-HIS3 downstream linking primer (SEQ ID NO: 10) | |

D-lactic acid dehydrogenase (D-LDH) specifically required for D-lactic acid production was introduced based on the strain having defects in the three genes such as ADH1, PDC1 and DLD1.

D-LDH was then cloned into a vector having restriction enzyme sites of *XhoI* and *SpeI* at 5' and 3' termini, respectively, in order for 1dhD derived from *lactobacillus plantarum* (*Lb. plantarum*) to be included between TEF1 promoter derived from *S. cerevisiae* and CYC1 terminator. In particular, the insert was prepared by double-digestion of *SacI*/*PvuII*, and the vector was blunt ended by Mungbean nuclease from the DNA fragment, which was double-digested from p-δ-neo into *BamHI*/*NotI.* Lastly, the vector was treated with SacI to thereby obtain a vector having a *SacI* sticky end and *BamHI* derived blunt end.

The construction of pTL573 vector was completed by the ligation of the obtained vector with the insert. The plasmid pTL573 contains the ldhD gene derived from *Lb. plantarum*, and it was designed so that it may include a random insertion of multiple copies of genes into partial domain of δ-sequence among retrotransposable element of *S. cerevisiae* CEN.PK2-1D pdc1Δ adh1Δ dld1A strain. For multiple insertion of a corresponding gene, DNA fragments capable of inducing single crossover on the δ-sequcnce were constructed by digesting plasmid pTL573 with SalI. By introducing the DNA fragments into a parent strain via transformation, a multiple colonies were obtained from YPD plate (1 % yeast extract, 2 % bacto-peptone, and 2 % glucose) at a maximum concentration of 5 mg/mL G418. Finally, it was confirmed that the thus-obtained strain, the *Lb. plantarum* derived D-LDH, was multiply inserted for the purpose of providing D-lactic acid-producing-ability, and was assigned CC02-0064 strain.

### Example 2: Preparation of mutant strains having decreased PDC5 activity

A mutant strain having substituted PDC5 promoter was prepared based on CC02-0064 strain prepared in Example 1. During the process, processes of cassette preparation and strain selection were conducted according to the method disclosed in Lee T. H. et al. (Development of reusable split URA3-marked knockout vectors for budding yeast, Saccharomyces cerevisiae. J. Microbiol. Biotechnol., 2006, 16:979-982).

Specifically, a total of five novel strains were prepared by substituting PDC5 promoter of the CC02-0064 strain with SCO1, SCO2, ACS1, IDP2, and FBA1 promoters, respectively, and subsequently, promoter-substituted cassettes were prepared using primers corresponding to nucleotide sequences of SEQ ID NOs: 11 to 36.

The primers used in the promoter substitution are summarized in Table 2 below.

**Table 2 Primers used for the preparation of promoter-substituted strains**

| Primers | 5'-> 3' sequence |
|---|---|
| F_PDC5_UP_676 (SEQ ID NO: 11) | GTCAGCATTGACACGTTCGATT |
| R_KlURA3-PDC5_UP (SEQ ID NO: 12) | |
| F_PDC5_UP-AL_KlURA3 (SEQ ID NO: 13) | |
| R_AL_KlURA3 (SEQ ID NO: 14) | GAGCAATGAACCCAATAACGAAATCTT |
| F_BR_KlURA3 (SEQ ID NO: 15) | CTTGACGTTCGTTCGACTGATGAG |
| R_PDC5_DOWN_522 (SEQ ID NO: 16) | CAAGTCAACCAAGTTAGCTGGC |
| R_SCO1p_BR_KlURA3 (SEQ ID NO: 17) | |
| F_SCO1p_500 (SEQ ID NO: 18) | CGTTCATGGTGACACCACGTCTATTAGGAGAGCCATTC |
| R_PDC5_DOWN_500-SCO1p (SEQ ID NO: 19) | |
| F_SCO1p-PDC5 DOWN 500 (SEQ ID NO: 20) | |
| R_SCO2p-BR_KlURA3 (SEQ ID NO: 21) | |
| F_SCO2p_500 (SEQ ID NO: 22) | CGTTCATGGTGACACGCTTGTTACTTATTCGATAACGC |
| R_PDC5_DOWN_500-SCO2p (SEQ ID NO: 23) | |
| F_SCO2p-PDC5_DOWN_500 (SEQ ID NO: 24) | |
| R_IDP2p-BR_K1URA3 (SEQ ID NO: 25) | |
| F_IDP2p_500 (SEQ ID NO: 26) | |
| R_PDC5_DOWN_500-IDP2p (SEQ ID NO: 27) | |
| F_IDP2p-PDC5_DOWN_500 (SEQ ID NO: 28) | |
| R_ACS1p-BR_K1URA3 (SEQ ID NO: 29) | |
| F_ACS1p_500 (SEQ ID NO: 30) | CGTTCATGGTGACACTGTGCACATACGTCCAGAATGAT |
| R_PDC5_DOWN_500-ACS1p (SEQ ID NO: 31) | |
| F_ACS1p-PDC5_DOWN_500 (SEQ ID NO: 32) | |
| R_FBA1p-BR_K1URA3 (SEQ ID NO: 33) | |
| F_FBA1p_500 (SEQ ID NO: 34) | |
| R_PDC5_DOWN_500-FBA1p (SEQ ID NO: 35) | |
| F_FBA1p-PDC5_DOWN_500 (SEQ ID NO: 36) | |

The thus-prepared novel strains were assigned CC02-0167, CC02-0168, CC02-0169, CC02-0170, and CC02-0174, respectively. The corresponding strains and their genetic traits are summarized in Table 3 below.

**Table 3 PDC5 promoter-mutated strains**

| Strains | Genetic Traits |
|---|---|
| CC02-0167 | CC02-0064 PDC5 promoter::KlURA3-SCO1 promoter |
| CC02-0168 | CC02-0064 PDC5 promoter::KlURA3-SCO2 promoter |
| CC02-0169 | CC02-0064 PDC5 promoter::KlURA3-ACS1 promoter |
| CC02-0170 | CC02-0064 PDC5 promoter::KlURA3-IDP2 promoter |
| CC02-0174 | CC02-0064 PDC5 promoter::KlURA3-FBA1 promoter |

### Example 3: Evaluation of Lactic acid fermentation for mutant strains having decreased PDC5 activity

An evaluation of lactic acid fermentation was conducted for the PDC5 promoter-mutated strains prepared in Example 2. In this regard, a specific medium was prepared for the evaluation of lactic acid fermentation.

Specifically, in order to prepare a synthetic complex media (SC media), a limiting medium for yeast, 0.67% yeast nitrogen base without amino acids serving as a base was mixed with amino acid dropout mix (Sigma) according to the protocol of the manufacturer, and added with the amino acids that were excluded in the base, as needed. In addition, 380 mg/L of leucine was added to the resultant, and uracil, tryptophan, and histidine were added at a concentration of 76 mg/L, respectively. 8% of glucose as a carbon source and 1% of CaCO₃ as a neutralizing agent were also added. The thus-prepared medium was used for the evaluation of lactic acid fermentation of the yeast strains.

Among the PDC5 promoter-mutated strains prepared in Example 2, the mutant strains substituted with a weaker promoter than the original PDC5 promoter failed to grow, whereas the mutant strains substituted with a stronger promoter showed improved growth. Specifically, the mutant strains substituted with promoters of SCO1, SCO2, IDP2 or ACS1, which are weaker promoters than PDC5 promoter, failed to grow, leaving the strains whose promoter was substituted with FBA1 promoter the only strains to be evaluated. The evaluation result of the lactic acid fermentation for CC02-0064 and CC02-0174 strains, which were measurable, is summarized in Table 4 below.

**Table 4 Evaluation of lactic acid fermentation for PDC5 promoter-mutated strains**

| Strain | 24 hours | | | 48 hours | | | |
|---|---|---|---|---|---|---|---|
| | OD | Glucose Consumed | Lactic acid | OD | Glucose Consumed | Lactic acid | Yield (%) |
| CC02-0064 | 3.9 | 15.0 | 10.9 | 8.7 | 63.4 | 41.6 | 65.7 |
| CC02-0174 | 5.7 | 25.0 | 19.8 | 9.4 | 69.9 | 47.3 | 67.7 |

As shown in the evaluation above, it was confirmed that, during the pathway promoting acetyl-CoA production, the strain where the wild-type PDC5 promoter was substituted with FBA1 promoter showed improved cell growth rate and lactic acid productivity thereof, compared to those of the original strain (CC02-0064). However, when the result of samples collected at 24 hours and 48 hours, respectively, were compared, it was confirmed that the improvements on the cell growth rate and the lactic acid productivity thereof according to the time were continued to reduce by a mere strengthening of a single PDC activity without strengthening ALD and ACS activities, which are involved in the subsequent acetyl-CoA producing pathway. According to Table 4, the improvement in the glucose consumption by the strengthening PDC activity was 10.3%, and the maximum lactic acid production concentration was 47.3 g/l. Accordingly, the overall improvement of the lactic acid productivity was 13.7%.

### Example 4: Preparation of a strain having a PDC5 gene defect.

In addition to the strain having a PDC1 gene defect and decreased PDC5 activity prepared in Example 2, a strain having a defect in PDC5 gene and decreased PDC1 activity was prepared to thereby confirm whether PDC pathway was attenuated in the corresponding strain.

Specifically, for the purpose of a PDC5 gene defect, the primers corresponding to nucleotide sequences of SEQ ID NOs: 37 to 40 were used to prepare PDC5 gene defect cassette based on the CC02-0064 strain. The defective strain was prepared by the same method described the literature of Example 1. The primers used in Example 4 are summarized in Table 5 below.

**Table 5 Primers used for the preparation of the strain having PDC5 defects**

| Primers | 5'-> 3' Sequence |
|---|---|
| F-ALPDC5-BamHI (SEQ ID NO: 37) | GAGCTCGGATCCAAGGAAATAAAGCAAATAACAATAACACC |
| R-ALPDC5-NotI (SEQ ID NO: 38) | ACCATGGCGGCCGCTTTGTTCTTCTTGTTATTGTATTGTGTTG |
| F-BRPDC5-SpeI (SEQ ID NO: 39) | GGATCCACTAGTGCTAATTAACATAAAACTCATGATTCAACG |
| R-BRPDC5-NcoI (SEQ ID NO: 40) | CAGCTGCCATGGTATTCTAAATAAGATGTAAGGCCTTGTAAT |

The thus-prepared strain having a PDC5 gene defect was assigned CC02-0450 (CC02-0064, pdc5Δ).

### Example 5: Preparation of PDC1 promoter-mutated strains based on the strain having a PDC5 defect

A strain having substituted PDC1 promoter was prepared based on the CC02-0450 strain prepared in Example 4. In this regard, a strain CC02-0451 (CC02-0450, PDC1p-PDC1), where the defect in PDC1 gene was recovered, was prepared to serve as a comparative group, and a strain CC02-0452 (CC02-0450, IDP1p-PDC1) having decreased PDC1 activity was prepared to serve as an experimental group.

Each strain was prepared in such a way that the vectors of PDC1p-PDC1-CYC1t and pRS406-IDP2p-PDC1-CYC1, which were constructed by cloning a target gene cassette into a pRS406 vector without a replication origin in the yeast, to be included in the strain.

Specifically, PCR was conducted using primers having nucleotide sequences of SEQ ID NOs: 41 and 42 with chromosomal DNA of the yeast serving as a template, to thereby obtain a product including PDC1 gene. Subsequently, a sequence of CYC1 terminator was obtained using primers having nucleotide sequences of SEQ ID NOs: 43 and 44. In addition, DNA fragments connecting PDC1 and CYC1 terminator were obtained via PCR using primers corresponding nucleotide sequences of SEQ ID NOs: 41 and 44 with the PDC1 and the CYC1 terminator sequences, respectively, serving as a template. A plasmid vector of pRS406-PDC1-CYC1t was obtained by treating DNA fragments of PDC1-CYC1 terminator and pRS406 vector with *SpeI* and *XhoI* restriction enzymes followed by ligation thereof. Meanwhile, for the introduction of the promoter domain into the thus-obtained plasmid vectors, plasmid vectors, into which promoters of PDC1 and IDP2 promoters were respectively incorporated, were obtained by a primer fusion of primers having nucleotide sequences of SEQ ID NOs: 45 and 46, and 47 and 48, respectively, via PCR using chromosomal DNA as a template. DNA fragments including each promoter and pRS406-PDC1-CYC1t plasmid were digested and ligated, to thereby prepare plasmid vectors of pRS406-PDC1p-PDC1-CYC1t and pRS406-IDP2p-PDC1-CYC1t, respectively, which are plasmid required for the yeast chromosomal insertion, designed such that the gene expression is controlled by PDC1 promoter and IDP2 promoter.

The primers used in Example 5 are summarized in Table 6.

**Table 6 Primers used for preparation of the strains having a PDC5 defect and decreased PDC1 activity**

| Primers | 5'-> 3' Sequence |
|---|---|
| F_PDC1 (SEQ ID NO: 41) | ATAACTAGTATGTCTGAAATTACTTTGGGTAAATATTT |
| R_PDC1 (SEQ ID NO: 42) | |
| F_CYC1t (SEQ ID NO: 43) | TACCAACGCTAAGCAATAAACAGGCCCCTTTTCCTTTGTCGAT |
| R_CYC1t (SEQ ID NO: 44) | ATACTCGAGGCAAATTAAAGCCTTCGAGCGTCC |
| F_PDC1p (SEQ ID NO: 45) | AAAGAGCTCCATGCGACTGGGTGAGCATATGTT |
| R_PDC1p (SEQ ID NO: 46) | ATAACTAGTTTTGATTGATTTGACTGTGTTATTTTGC |
| F_IDP2p (SEQ ID NO: 47) | AAAGAGCTCACGTCTATCTATTTATTTTTATAACTCC |
| R_IDP2p (SEQ ID NO: 48) | ATAACTAGTTACGATTTTATATATATACGTACGTTAC |

The two thus-prepared plasmid vectors were digested by StuI, respectively, and inserted into the strains immediately. The final strains were assigned CC02-0451 (CC02-0450, PDC1p-PDC1) and CC02-0452 (CC02-0450, IDP2p-PDC1), respectively. The thus-prepared strains and their genetic traits are summarized in Table 7.

**Table 7 Strains having PDC5 defect and decreased PDC1 activity**

| Strains | Genetic Traits |
|---|---|
| CC02-0450 | CC02-0064 pdc5Δ |
| CC02-0451 | CC02-0450 PDC1p-PDC1-CYC1t |
| CC02-0452 | CC02-0450 IDP2p-PDC1-CYC1t |

### Example 6: Preparation of strains having double or triple defects in PDC genes

Strains having a single defect in PDC1 gene, a double defect in PDC1 and PDC5 genes, and a triple defect in PDC1, PDC5, and PDC6 genes were intended to be prepared from PDC family genes. CC02-0064 strain prepared in Example 1 was used as a strain having a single defect in PDC 1 gene. A cassette for PDC5 defect was prepared using primers corresponding nucleotide sequences of SEQ ID NOs: 49 to 56, and inserted into CC02-0064 to prepare a strain having double defects in PDC1 and PDC5 genes. Subsequently, the thus-prepared strain was assigned CC02-0256. In addition, a strain having a triple defect in PDC1, PDC5, and PDC6 genes was prepared based on the strain having double defects in PDC1 and PDC5 genes using primers corresponding to nucleotide sequence of SEQ ID NOs: 57 to 64, and was assigned CC02-0257.

The defect cassette preparation and strain selection process were conducted by the same method described in the literature disclosed in Example 1. The primers used in Example 6 are summarized in Table 8.

**Table 8 Primers used for preparation of the strains having double or triple defects in PDC genes**

| Primers | 5' -> 3' Sequence |
|---|---|
| F_BamHI-PDC5_UP (SEQ ID NO: 49) | CGGGATCCAGGCCAAGGAAATAAAGCAAATAACAA |
| R_NotI-PDC5_UP (SEQ ID NO: 50) | |
| F_BamHI-PDC5_DOWN (SEQ ID NO: 51) | CGGGATCCGCTAATTAACATAAAACTCATGATTCAA |
| R_NotI-PDC5_DOWN (SEQ ID NO: 52) | |
| F_PDC5_UP (SEQ ID NO: 53) | AGGCCAAGGAAATAAAGCAAATAACAA |
| R_AL_KlURA3 (SEQ ID NO: 54) | GAGCAATGAACCCAATAACGAAATCTT |
| F_BR_KlURA3 (SEQ ID NO: 55) | CTTGACGTTCGTTCGACTGATGAG |
| R_PDC5_DOWN (SEQ ID NO: 56) | TATTCTAAATAAGATGTAAGGCCTTGTA |
| F_BamHI-PDC6_UP (SEQ ID NO: 57) | CGGGATCCTGTTATAGAGTTCACACCTTATTCACA |
| R_NotI-PDC6_UP (SEQ ID NO: 58) | |
| F_BamHI-PDC6_DOWN (SEQ ID NO: 59) | CGGGATCCGCCATTAGTAGTGTACTCAAACGAAT |
| R_NotI-PDC6_DOWN (SEQ ID NO: 60) | |
| F_PDC6_UP (SEQ ID NO: 61) | TGTTATAGAGTTCACACCTTATTCACA |
| R_AL_KlURA3 (SEQ ID NO: 62) | GAGCAATGAACCCAATAACGAAATCTT |
| F_BR_KlURA3 (SEQ ID NO: 63) | CTTGACGTTCGTTCGACTGATGAG |
| R_PDC6_DOWN (SEQ ID NO: 64) | GATGCAGAATGAGCACTTGTTATTTAT |

The thus-prepared strains and their genetic traits are summarized in Table 9.

**Table 9 Strains having double or triple defects in PDC genes**

| Strains | Genetic traits |
|---|---|
| CC02-0256 | CC02-0064 pdc5Δ |
| CC02-0257 | CC02-0256 pdc6Δ |

### Example 7: Preparation of ALD and ACS1 overexpressing strains

For the preparation of ALD and ACS1 overexpressing strains, ALD2, ALD3, and ACS1 overexpressing plasmids were prepared.

Specifically, an open reading frame (ORF) of ALD2 was prepared using primers corresponding nucleotide sequences of SEQ ID NOs: 65 and 66, an ORF of ALD3 was prepared using primers corresponding nucleotide sequences of SEQ ID NOs: 67 and 68, and an ORF of ACS1 was prepared using primers corresponding nucleotide sequences of SEQ ID NOs: 69 and 70. In addition, p415ADH-ALD2, p415ADH-ALD3, p414ADH-ACS1 and p416ADH-ACS1, which are p414ADH, p415ADH and p416ADH plasmid-based recombinant vectors, were prepared by *Spe*I, *Xho*I or *EcoR*I restriction enzymes. The primers used in Example 7 are summarized in Table 10 below.

**Table 10 Primers used for the preparation of ALD and ACS1 overexpressing strains**

| Primers | 5'-> 3' Sequence |
|---|---|
| F_SpeI_ALD2 (SEQ ID NO: 65) | |
| R_XhoI_ALD2 (SEQ ID NO: 66) | |
| F_SpeI_ALD3 (SEQ ID NO: 67) | |
| R_XhoI_ALD3 (SEQ ID NO: 68) | |
| F_ACS1 (SEQ ID NO: 69) | |
| R_EcoRI_ACS1 (SEQ ID NO: 70) | |

The thus-prepared recombinant plasmids were introduced into the strains including CC02-0064, CC02-0168, CC02-0170, CC02-0256, CC02-0257, CC02-0451, and CC02-0452 via a yeast transformation by p415ADH-ALD2, p414ADH-ACS1 combination, p415ADH-ALD3, p414ADH-ACS1 combination, p415ADH-ALD2, p416ADH-ACS1 combination, or p415ADH-ALD3, p416ADH-ACS1 combination. However, no transformant was obtained in the CC02-0257 strain having triple defects in PDC genes where no PDC activity was exhibited.

The thus-prepared strains and their genetic traits are summarized in Table 11.

**Table 11 ALD and ACS overexpressing strains**

| Strains | Genetic Traits |
|---|---|
| CC02-0225 | CC02-0064 p415ADH, p416ADH |
| CC02-0226 | CC02-0064 p415ADH-ALD2, p416ADH-ACS1 |
| CC02-0227 | CC02-0064 p415ADH-ALD3, p416ADH-ACS1 |
| CC02-0356 | CC02-0168 p414ADH, p415ADH |
| CC02-0275 | CC02-0168 p414ADH-ACS1, p415ADH-ALD2 |
| CC02-0276 | CC02-0168 p414ADH-ACS1, p415ADH-ALD3 |
| CC02-0357 | CC02-0170 p414ADH, p415ADH |
| CC02-0277 | CC02-0170 p414ADH-ACS1, p415ADH-ALD2 |
| CC02-0278 | CC02-0170 p414ADH-ACS1, p415ADH-ALD3 |
| CC02-0444 | CC02-0256 p415ADH, p416ADH |
| CC02-0361 | CC02-0256 p415ADH-ALD2, p416ADH-ACS1 |
| CC02-0362 | CC02-0256 p415ADH-ALD3, p416ADH-ACS1 |
| CC02-0453 | CC02-0451 p414ADH, p415ADH |
| CC02-0454 | CC02-0451 p414ADH-ACS1, p415ADH-ALD2 |
| CC02-0455 | CC02-0451 p414ADH-ACS1, p415ADH-ALD3 |
| CC02-0456 | CC02-0452 p414ADH, p415ADH |
| CC02-0457 | CC02-0452 p414ADH-ACS1, p415ADH-ALD2 |
| CC02-0458 | CC02-0452 p414ADH-ACS1, p415ADH-ALD3 |

### Example 8: Evaluation of lactic acid fermentation for the yeast strains

An evaluation of lactic acid fermentation-ability for the ALD and ACS1 overexpressing strains, prepared in Example 7, was conducted.

Specifically, the yeast was inoculated into each flask containing 25 ml of the medium, prepared in Example 3 for the purpose of lactic acid fermentation evaluation and was cultured under aerobic condition at 30°C for 71 hours. The amount of D-type lactic acid present in the fermented broth was analyzed, and an enzymatic analysis (Acetic acid, R-Biopharm, Germany) was conducted to determine the amount of acetic acid present therein.

The above experiment results are summarized in Table 12 below.

**Table 12 Evaluation of the growth rate, lactic acid fermentation, by-products, and production yield, etc., for the ALD and ACS overexpressing strains**

| Strains | Final OD | Initial glucose (g/L) | Residual glucose (g/L) | Acetate (g/L) | D-lactic acid (g/L) | Yield (g/g) | Productivity (g/l·h) |
|---|---|---|---|---|---|---|---|
| CC02-0225 (ref. example) | 9.3 | 88 | 10 | 2.87 | 41.1 | 0.53 | 0.579 |
| CC02-0226 (ref. example) | 9.3 | 83 | 10.5 | 2.91 | 42.4 | 0.59 | 0.597 |
| CC02-0227 (ref. example) | 10.1 | 84 | 9.5 | 2.91 | 41.8 | 0.56 | 0.589 |
| CC02-0356 (ref. example) | 6.9 | 88 | 26 | 0.02 | 27.6 | 0.45 | 0.389 |
| CC02-0275 | 11.6 | 88 | 11.5 | 0.01 | 47.6 | 0.62 | 0.670 |
| CC02-0276 | 10.6 | 88 | 11 | 0.01 | 46.8 | 0.61 | 0.659 |
| CC02-0357 (ref. example) | 12.2 | 88 | 13 | 0.04 | 38.1 | 0.51 | 0.537 |
| CC02-0277 | 17.8 | 88 | 1 | 0.03 | 58.6 | 0.67 | 0.825 |
| CC02-0278 | 18.8 | 88 | 0 | 0.02 | 56.9 | 0.66 | 0.801 |
| CC02-0453 (ref. example) | 9.8 | 88 | 8.5 | 2.2 | 38.9 | 0.49 | 0.548 |
| CC02-0454 (ref. example) | 10.2 | 88 | 8.1 | 2.4 | 39.5 | 0.49 | 0.556 |
| CC02-0455 (ref. example) | 9.2 | 88 | 8.8 | 2.1 | 40 | 0.51 | 0.563 |
| CC02-0456 (ref. example) | 12 | 88 | 10.1 | 0.02 | 38.5 | 0.49 | 0.542 |
| CC02-0457 | 18.1 | 88 | 0 | 0.02 | 55.8 | 0.63 | 0.786 |
| CC02-0458 | 18.5 | 88 | 0 | 0.02 | 56.5 | 0.64 | 0.800 |

As verified in Table 12, the strains having decreased PDC5 activity by IDP2 promoter or SCO2 promoter had a dramatic reduction in the accumulation of the acetate, a by-product, *i.e.,* little detection of acetate was confirmed, compared to the strain with normal PDC5 activity. In such case, the final cell concentration of the strains, in which the activities of ALD and ACS were not increased, tended to decrease according to the PDC5 promoter substitutions. On the contrary, the strains, where the PDC5 expression was reduced and the activities of ALD and ACS were increased, showed an increase in the final cell concentration. Accordingly, the improvement in the cell growth was confirmed. According to the present invention, the strains of CC02-0277 and CC02-0278 having increased ALD and ACS activities prepared based on the CC02-0170 strain, where PDC5 promoter was substituted with IDP2, showed improved growth rate, D-lactic acid concentration of production, production yield thereof, and fermentation productivity as increasing in the ALD and ACS activities.

In summary, the strain where PDC5 was substituted with a weak expression of IDP2 had a reduction in acetate accumulation, and the final OD was 1.3 times higher compared to the strain exhibiting normal expression of PDC5. In addition, it was confirmed that, when ALD and ACS were co-expressed under the control of the ADH1 promoter, the glucose consumption and the rate thereof were increased, and finally, the percentage yield was increased from 56% or 59% to 66% or 67%, respectively, showing improved yield.

Specifically, by comparing the two kinds of promoters applied to the weak expression of PDC5, it was confirmed that, the lactic acid productivity was improved in both strains having SCO2 promoter and IDP2 promoter, respectively. However, the strain having IDP2 promoter may be considered as the most optimized form of a strain in terms of the overall cell concentration, the glucose consumption, and the rate thereof.

### Example 9: Evaluation of lactic acid fermentation for the yeast strain having double defects in PDC1 and PDC5 genes, and increase in ALD and ACS activities

Since the effects of the cell growth and the yield improvements resulted from the decreased PDC5 activity have been clearly confirmed, an evaluation was undertaken to determine the effects of additional PDC gene defect in lactic acid production. The evaluation method for each strain was identical to the method described in Example 8, and the culturing was conducted for 74 hours.

The thus-obtained experiment results are summarized in Table 13 below.

**Table 13 The evaluation results of lactic acid fermentation for the strains having double defects in PDC1 and PDC5 genes**

| Strains | Final OD | Initial Glucose (g/L) | Residual Glucose (g/L) | Acetate (g/L) | D-lactic acid (g/L) | Yield (g/g) | Productivity (g/l·h) |
|---|---|---|---|---|---|---|---|
| CC02-0444 | 3.2 | 78.5 | 52 | 0.10 | 20.8 | 78.5 | 0.281 |
| CC02-0361 | 3.9 | 78.5 | 49 | 0.05 | 25.4 | 86.3 | 0.343 |
| CC02-0362 | 3.8 | 78.5 | 51 | 0.08 | 22.8 | 82.8 | 0.308 |

As confirmed in Table 13, the acetate concentration was clearly reduced in the strains having double defects in PDC1 and PDC5 genes, however, a reduction in the D-lactic acid concentration of production was also observed due to the reduction in the cell growth and the glucose consumption. In addition, the strain where the PDC pathway is almost inactivated, which was resulted from the double defects in PDC1 and PDC5 genes, did not have any improvement in the cell growth, the glucose consumption, and the productivity thereof, although the strain exhibited increased activities ofALD and ACS.

### Example 10: Evaluation of lactic acid fermentation for the strains, where PDC pathway is attenuated, using sucrose

For the purpose of a fermentation evaluation using sucrose, the lactic acid-producing yeast strains, where PDC pathway is attenuated, the identical strains evaluated in Example 8 and 9 were used to confirm the effect of the lactic acid production. In this regard, sucrose was employed as a carbon source instead of glucose. The evaluation method was performed in the same manner as Example 8.

The thus-obtained experiment results are summarized in Table 14 below.

**Table 14 The evaluation results of lactic acid fermentation for the strains having double defects in PDC 1 and PDC5 genes or decreased PDC5 activity**

| | | | | | | | |
|---|---|---|---|---|---|---|---|
| Strains | Final OD | Initial Glucose (g/L) | Residual Glucose (g/L) | Acetate (g/L) | D-lactic acid (g/L) | Yield (g/g) | Productivity (g/l·h) |
| CC02-0225 (ref. example) | 5.15 | 91.5 | 27.5 | 1.95 | 25.19 | 39.36 | 0.34 |
| CC02-0226 (ref. example) | 6.9 | 91.5 | 15 | 1.92 | 30.89 | 40.38 | 0.42 |
| CC02-0227 (ref. example) | 6.18 | 91.5 | 15 | 1.98 | 29.59 | 38.68 | 0.40 |
| CC02-0356 (ref. example) | 1.6 | 91.5 | 26.75 | 0.02 | 11.72 | 18.11 | 0.16 |
| CC02-0275 | 1.88 | 91.5 | 21.75 | 0.02 | 13.79 | 19.77 | 0.19 |
| CC02-0276 | 2.2 | 91.5 | 18.25 | 0.01 | 16.04 | 21.9 | 0.22 |
| CC02-0357 (ref. example) | 2.78 | 91.5 | 22 | 0.03 | 17.08 | 24.58 | 0.23 |
| CC02-0277 | 12.15 | 91.5 | 7.75 | 0.02 | 44.65 | 53.31 | 0.60 |
| CC02-0278 | 11.88 | 91.5 | 6.25 | 0.01 | 43.84 | 51.43 | 0.60 |
| CC02-0444 | 2.45 | 91.5 | 37 | 0.02 | 21.94 | 40.25 | 0.30 |
| CC02-0361 | 2.5 | 91.5 | 18.25 | 0.02 | 21.73 | 29.66 | 0.29 |
| CC02-0362 | 3.08 | 91.5 | 15.25 | 0.02 | 24.92 | 32.67 | 0.34 |

The use of sucrose instead of glucose for the strains, used in the same manner as in Examples 8 and 9, allowed for improved effects of the growth and fermentation yield by increasing the activities of ALD and ACS in the strains where PDC pathway is attenuated, showing the same pattern of results as the strains in Example 8 where glucose was employed as a carbon source. Accordingly, the present invention confirms that the improved effects of fermentation yield and growth due to the decreased PDC activity and increased ALD and ACS activities, which were confirmed in the present invention, are not limited to the type of sugar used.

To summarize the above results, it was confirmed that, when the strains were mutated in such a way that PDC pathway was attenuated, and the activities of ALD and ACS were improved compared to that of the non-mutated strains, the lactic acid production was increased, and the growth rate thereof was maintained simultaneously.
## Claims

1. An isolated *Saccharomyces cerevisiae* microorganism having enhanced productivity of lactic acid, wherein the microorganism is modified so that:
a) i) the activity of PDC1 is inactivated and the activity of PDC5 is decreased, or ii) the activity of PDC1 is decreased and the activity of PDC5 is inactivated; and
b) the activities of aldehyde dehydrogenase (ALD) and acetyl-CoA synthetase (ACS) of the microorganism are enhanced,
wherein the activity is enhanced by an insertion of a plasmid containing the genes of an enzyme, an increase in the number of gene copies encoding an enzyme on a chromosome, or an increase in an enzyme activity caused by a substitution or modification, or a mutation of a promoter sequence of an enzyme gene;
wherein the activity is decreased by substitution or modification of a promoter sequence of the enzyme gene, or mutation into an enzyme having decreased activity caused by a substitution or modification of the enzyme; and
wherein the activity is inactivated by a partial gene deletion or a whole gene deletion, an insertion of a foreign-derived gene into the relevant gene, substitution or modification of a gene promoter sequence of the enzyme, or a mutation into an inactive-enzyme having a loss in its original functions caused by a substitution or modification of the enzyme.

2. The microorganism according to Claim 1, wherein the aldehyde dehydrogenase is at least one selected from the group consisting of ALD2 and ALD3, and the acetyl-CoA synthetase is ACS1.

3. The microorganism according to Claim 1, wherein alcohol dehydrogenase (ADH) is further inactivated.

4. The microorganism according to Claim 1, wherein D-lactic acid dehydrogenase (DLD) is further inactivated.

5. The microorganism according to Claim 1, wherein ADH1, PDC1 and DLD1 are inactivated, and D-lactate dehydrogenase (D-LDH) is further introduced.

6. A method for producing lactic acid comprising:
a) culturing the microorganism according to any one of Claims 1 to 5 in the culture medium; and
b) recovering lactic acid from the culture medium or the microorganism in step a).

## Patentansprüche

1. Isolierter *Saccharomyces cerevisiae*-Mikroorganismus mit erhöhter Milchsäureproduktivität, wobei der Mikroorganismus modifiziert ist, so dass:
a) i) die Aktivität von PDC1 inaktiviert ist und die Aktivität von PDC5 verringert ist, oder ii) die Aktivität von PDC1 verringert ist und die Aktivität von PDC5 inaktiviert ist; und
b) die Aktivitäten von Aldehyd-Dehydrogenase (ALD) und Acetyl-CoA-Synthetase (ACS) des Mikroorganismus erhöht sind,
wobei die Aktivität durch eine Insertion eines Plasmids, das die Gene eines Enzyms enthält, eine Erhöhung der Zahl von Genkopien, die für ein Enzym auf einem Chromosom kodieren oder eine Erhöhung in einer Enzymaktivität, verursacht durch eine Substitution oder Modifikation, oder eine Mutation einer Promotorsequenz eines Enzymgens erhöht ist;
wobei die Aktivität durch Substitution oder Modifikation einer Promotorsequenz des Enzymgens oder eine Mutation in einem Enzym mit verringerter Aktivität, verursacht durch eine Substitution oder Modifikation des Enzyms, verringert ist; und
wobei die Aktivität durch eine partielle Gendeletion oder eine vollständige Gendeletion, eine Insertion eines Gens fremder Abstammung in das relevante Gen, Substitution oder Modifikation einer Genpromotorsequenz des Enzyms oder eine Mutation zu einem inaktiven Enzym mit einem Verlust seiner ursprünglichen Funktionen, verursacht durch eine Substitution oder eine Modifikation des Enzyms, inaktiviert ist.

2. Mikroorganismus nach Anspruch 1, wobei die Aldehyd-Dehydrogenase wenigstens eine ist, ausgewählt aus der Gruppe, bestehend aus ALD2 und ALD3, und die Acetyl-CoA-Synthetase ACS1 ist.

3. Mikroorganismus nach Anspruch 1, wobei die Alkoholdehydrogenase (ADH) weiter inaktiviert ist.

4. Mikroorganismus nach Anspruch 1, wobei die D-Milchsäure-Dehydrogenase (DLD) weiter inaktiviert ist.

5. Mikroorganismus nach Anspruch 1, wobei ADH1, PDC1 und DLD1 inaktiviert sind und die ferner D-Lactat-Dehydrogenase (D-LDH) eingebracht ist.

6. Verfahren zur Herstellung von Milchsäure, umfassend:
a) Kultivieren des Mikroorganismus nach einem der Ansprüche 1 bis 5 in dem Kulturmedium;
und
b) Gewinnen der Milchsäure aus dem Kulturmedium oder dem Mikroorganismus in Schritt a).

## Revendications

1. Microorganisme isolé de *saccharomyces cerevisiae* ayant une productivité améliorée d'acide lactique, le microorganisme étant modifié de manière que:
a) i) l'activité de PDC1 est inactivée et l'activité de PDC5 est réduite, ou ii) l'activité de PDC1 est réduite et l'activité de PDC5 est inactivée; et
b) les activités d'aldéhyde-déshydrogénase (ALD) et d'acétyl-coA-synthétase (ACS) du microorganisme sont améliorées,
l'activité étant améliorée par une insertion d'un plasmide contenant des gènes d'une enzyme, une augmentation du nombre de copies du gène codant pour une enzyme sur un chromosome, ou une augmentation dans une activité d'enzyme causée par une substitution ou une modification, ou une mutation d'une séquence promoteur d'un gène d'enzyme;
l'activité étant réduite par substitution ou modification d'une séquence promoteur du gène d'enzyme, ou la mutation dans une enzyme ayant une activité réduite causée par une substitution ou une modification de l'enzyme; et
l'activité étant inactivée par une délétion partielle du gène ou une délétion totale du gène, une insertion d'un gène étranger dérivé (*foreign-derived gene*) dans le gène pertinent, une substitution ou une modification d'une séquence promoteur de gène de l'enzyme, ou une mutation dans une enzyme inactive ayant une perte dans ses fonctions originales causée par une substitution ou une modification de l'enzyme.

2. Microorganisme selon la revendication 1, l'aldéhyde-déshydrogénase étant au moins une choisie parmi le groupe constitué d'ALD2 et ALD3, et l'acétyl-coA-synthétase étant ACS1.

3. Microorganisme selon la revendication 1, l'alcool-déshydrogénase (ADH) étant inactivée davantage.

4. Microorganisme selon la revendication 1, l'acide d-lactique déshydrogénase (DLD) étant inactivée davantage.

5. Microorganisme selon la revendication 1, l'ADH1, la PDC1 et la DLD1 étant inactivées, et la d-lactate déshydrogénase (D-LDH) étant introduite davantage.

6. Procédé de production d'acide lactique, comportant:
a) cultiver le microorganisme selon l'une quelconque des revendications 1 à 5 dans le milieu de culture;
et
b) récupérer de l'acide lactique à partir du milieu de culture ou du microorganisme dans l'étape a).
